Europäisches Patentamt

European Patent Office

Office européen des brevets

(19)

(11) Veröffentlichungsnummer: **0 541 058 A1**

## (12) EUROPÄISCHE PATENTANMELDUNG

(21) Anmeldenummer: **92118880.1**

(22) Anmeldetag: **04.11.92**

(51) Int. Cl.5: **C12N 9/50**, C07K 15/00, C12N 15/57, A61K 37/64

(30) Priorität: **06.11.91 DE 4136443**

(43) Veröffentlichungstag der Anmeldung:
**12.05.93 Patentblatt 93/19**

(84) Benannte Vertragsstaaten:
**AT BE CH DE DK ES FR GB GR IE IT LI LU MC NL PT SE**

(71) Anmelder: **BOEHRINGER INGELHEIM INTERNATIONAL GmbH**
**Postfach 200**
**W−6507 Ingelheim am Rhein(DE)**

(72) Erfinder: **Ahorn, Horst, Dr.**
**Wienerstrasse 5**
**A−2484 Weigelsdorf(AT)**
Erfinder: **Maurer−Fogy, Ingrid, Dr.**
**Lindauergasse 35**
**A−1238 Wien(AT)**
Erfinder: **Sommergruber, Wolfgang, Dr.**
**Stoesslgasse 6**
**A−1130 Wien(AT)**
Erfinder: **Zöphel, Andreas, Dr.**
**Schubertstrasse 100**
**A−3040 Neulengbach(AT)**
Erfinder: **Blaas, Dieter, Dr.**
**Liechtensteinstrasse 119/13**
**A−1190 Wien(AT)**
Erfinder: **Küchler, Ernst, Prof. Dr.**
**Görgengasse 23/6/26**
**A−1190 Wien(AT)**
Erfinder: **Liebig, Hans−Dieter, Dr.**
**Dörfelgasse 23/6/26**
**A−1120 Wien(AT)**
Erfinder: **Skern, Timothy, Dr.**
**Lerchenfelder Gürtel 45/15**
**A−1160 Wien(AT)**

(54) **Expression der reifen Proteinase 2A, ihre partielle Reinigung und Bereitstellung inhibitorisch wirkender Substrate.**

(57) Die Erfindung betrifft die Expression der reifen HRV Proteinase 2A, ihre partielle Reinigung und Bereitstellung inhibitorisch wirkender Substrate.

Gegenstand der Erfindung ist die Bereitstellung der reifen HRV2 Proteinase 2A, ihre partielle Reinigung und die Bereitstellung inhibitorisch wirksamer Substrate.

Viele tierische und pflanzliche Viren benötigen während ihres Replikationszyklus den Einsatz von viral – kodierten Proteinasen. Die Picornaviren, eine Familie von bedeutenden human – und animalpathogenen Viren, sind zum Beispiel vollständig von einem proteolytischen Prozessieren abhängig. Die bei der Replikation involvierten proteolytischen Enzyme sind hoch Substrat – spezifisch und erkennen meist eine Spaltregion – also ein strukturell determiniertes Erkennungsmerkmal – und weniger ein genau definiertes Aminosäurepaar, wie es üblicherweise als Erkennungssequenz dient. Einen generellen Überblick zu dieser Thematik geben H.G. Kräusslich und E. Wimmer (1988, Ann. Rev. Biochem. 57, 701 – 751) und Kay, J. und Dunn, B. M. (1990, Biochim. Biophys. Acta 1048, 1 – 18).

Die viralen Proteinasen stellen aufgrund ihrer Substratspezifität und ihres katalytischen Mechanismus einen guten therapeutischen Angriffspunkt dar (Johnston, M. I. et al., 1989, Trends Pharmacol. Sci. 10, 305 – 307). Durch die Bereitstellung der dreidimensionalen Struktur der Proteinasen des Rous Sarcoma Virus (Leis, J. et al., 1990, ASM – News 56, 77 – 81) und von HIV I (Navia, M. A. et al., 1989, Nature 337, 615 – 620; Miller, M. et al., 1989, Science 246, 1149 – 1152) ist es möglich, ein Computer – unterstütztes molekulares "designing" hoch spezifischer Inhibitoren durchzuführen (Meek, T. D. et al., 1990, Nature 343, 90 – 92). Spezifische Proteinaseinhibitoren könnten somit neue antivirale Substanzen darstellen, welche beispielsweise gegen Viren gerichtet sind, gegen die die Entwicklung eines Vakzins aus rein technischen Gründen nicht möglich ist; z.B. sind bei Rhinoviren derzeit weit über 115 nicht miteinander kreuz reagierende Serotypen bekannt, wovon 90 bereits als definierte Serotypen klassifiziert wurden (Cooney, M. K. et al., 1982, Infect. Immun. 37, 642 – 647).

Proteolytisches Prozessieren des Polyproteins (Jacobson, M.F. und Baltimore, D., 1968, Proc. Natl. Acad. Sci. 83, 5392) durch viral kodierte Proteinasen ist bevorzugt bei ( + )Strang RNA Viren (Hellen, C.U.T. et al., 1989, Biochemistry 28, 9881 – 9890) und Retroviren anzutreffen (Skalka, A. M., 1989, Cell 56, 911 – 913). Mit Hilfe von molekularbiologischen Studien, Sequenzvergleichen und Röntgenstrukturanalysen stellte sich heraus, daß die von Viren kodierten Proteinasen zwei Gruppen von proteolytischen Enzymen zuge – ordnet werden können, nämlich den Pepsin – ähnlichen Aspartat – Proteinasen (Meek, T. D. et al., 1989, Proc. Natl. Acad. Sci. 86, 1841 – 1845) und den Cystein – Proteinasen mit Trypsin – ähnlicher Proteinket – tenfaltung (Bazan, J. F. and Fletterick, R. J., 1988, Proc. Natl. Acad. Sci. 85, 7872 – 7876). Nicht immer sind beim proteolytischen Prozessieren des viralen Polyproteins ausschließlich viral – kodierte Proteinasen be – teiligt, so z.B. sind bei der Reifungsspaltung des Polyproteins des "Yellow Fever Virus", welches zu den Flaviviridae gehört, zelluläre Proteinasen beteiligt (Ruiz – Linares, A. et al., 1989, J. Virol. 63, 4199 – 4209).

Die Substratspezifität der viralen Enzyme konnte mittels detaillierter Studien wie Punktmutationsanaly – sen, Spaltung von Peptidsubstraten "in vitro" und Aminosäure – Sequenzierung der nativen Spaltprodukte näher analysiert werden. Dabei stellte sich heraus, daß, wie oben bereits erwähnt, weniger die Spaltstelle selbst als einige Positionen "up" – oder "downstream" eine tragende Rolle bei der Spaltstellenerkennung spielen. Jene Sequenz – Heterogenität der Spaltsignale bzw. deren unmittelbarer Umgebung führt jedoch zu einer Art "Hierarchie" der Spaltereignisse im Polyprotein (Kräusslich, H. G. et al., 1989, Proc. Natl. Acad. Sci. 86, 807 – 811; Pichuantes, S. et al., 1989, PROTEINS: Structure, Function and Genetics 6, 324 – 337; Darke, P. L. et al., 1989, J. Biol. Chem. 264, 2307 – 2312; Nicklin, M. J. H. et al., 1988, J. Virol. 62, 4586 – 4593; Libby, R. T. et al., 1988, Biochemistry 27, 6262 – 6268; Sommergruber, W. et al., 1989, Virology 169, 68 – 77; Skern, T. et al., 1991, Virology, 181, 46 – 54). Die Variation der einzelnen Spaltregionen im Polyprotein erlaubt so eine genau determinierte Abfolge von kinetisch "günstigen" bis zu kinetisch "ungünstigen" Spaltungen, die in weiterer Folge eine differenzierte Proteolyse der einzelnen Spaltprodukte ermöglicht. Dadurch kommt den viralen Proteinasen eine Art von regulatorischem Potential während des viralen Replikationszyklus zu. Das Prinzip der Erkennung einer spezifischen Sekundärstruktur im Spaltstel – lenbereich ist nicht auf Picornaviren beschränkt, sondern dürfte ein allgemeines Prinzip der viralen Proteinasen darstellen. So werden diese Eigenschaften z.B. im Adenovirussystem (Webster, A. et al., 1989, J. Gen. Virol. 70, 3225 – 3234; Webster, A. et al., 1989, J. Gen. Virol. 70, 3215 – 3223) und in pflanzenviralen Systemen ebenso angetroffen (Carrington, J.C. und Dougherty, W.G., 1988, Proc. Natl. Acad. Sci. 85, 3391 – 3395; Dougherty, W.G. et al., 1988, EMBO J. 7, 1281 – 1288).

Kaum ein anderes virales System ist bei seiner Regulation des Infektionsablaufes dermaßen von einer kontrolliert limitierten Proteolyse abhängig, wie das der Picornaviridae. Diese Virenfamilie läßt sich in 4 verschiedene Genera unterteilen: Entero – , Rhino – , Aphto – und Cardioviren. Rhinoviren sind wie alle anderen Vertreter dieser Virenfamilie einzelsträngige ( + )RNA – Viren (Cooper, P. D. et al., 1978, Interviro – logy 10, 165 – 180; MacNaughton, M. R., 1982, Current Top. Microbiol. Immunol. 97, 1 – 26). Sie sind weit verbreitet, befallen den oberen respiratorischen Trakt des Menschen und verursachen akute Infektionen, die zu Schnupfen, Husten, Heiserkeit etc. führen und allgemein als Erkältungen bezeichnet werden (Stott, E. J.

und Killington, R. A., 1972, Ann. Rev. Microbiol. 26, 503 – 524). Infektionen durch Rhinoviren zählen zu den häufigsten Erkrankungen des Menschen. Die Krankheit verläuft zwar meist harmlos, dennoch kommt es – bedingt durch eine vorübergehende Schwächung des Organismus – zu Sekundärinfektionen durch andere Viren oder Bakterien, die dann unter Umständen schwere Erkrankungen zur Folge haben. Von den insgesamt ca. 115 verschiedenen, bekannten Serotypen von humanen Rhinoviren sind bis jetzt 5 Serotypen (HRV 1B, 2, 9, 14 und 89) kloniert und komplett sequenziert worden: Deutsche Patentanmeldung P 35 05 148.5; Skern, T. et al., 1985, Nucleic Acids Res. 13, 2111 – 2126; Düchler, M. et al., 1987, Proc. Natl. Acad. Sci. USA 84, 2605 – 2609; Stanway, G. et al., 1984, Nucleic Acids Res. 12, 7859 – 7877; Callahan, P. L. et al., 1985, Proc. Natl. Acad. Sci. USA 82, 732 – 736; Hughes, R. et al., 1988, J. Gen. Virol. 69, 49 – 58, Lecki; G.W., 1988, Ph.D. thesis, University of Reading).

Die genomische einzelsträngige ( + )RNA der Rhinoviren wird kurz nach der Infektion durch Abspaltung des an das 5'Ende gebundenen Oligopeptids VPg modifiziert und dient in der Folge als mRNA für die Synthese eines Polyproteins, das den gesamten durchgehenden Leserahmen der Nukleinsäuresequenz umfaßt (Butterworth, B. E., 1973, Virology 56, 439 – 453; Mc Lean, C. und Rueckert, R. R., 1973, J. Virol. 11, 341 – 344; Mc Lean, C. et al., 1976, J. Virol. 19, 903 – 914; Agol, V.I., 1980, Prog. med. Virol. 26, 119 – 157; Putnak, J.R. und Phillips, B.A., 1981, Microbiol. Rev. 45, 287 – 315). Die reifen viralen Proteine entstehen ausschließlich durch proteolytische Spaltung aus diesem Polyprotein, wobei – zumindest bei Entero – und Rhinoviren – die dabei wirksamen Proteinasen selbst Bestandteil dieses Polyproteins sind. Das Prozes – sieren erfolgt in 3 Stufen (Palmenberg, A., 1987, J. Cell. Biochem. 33, 191 – 198; Kräusslich, H.G. und Wimmer, E., 1988, loc. cit.):

1.) Die Primärspaltung: Trennung der Kapsidvorläufer von der wachsenden Polypeptidkette;

2.) die Sekundärspaltung: Prozessieren struktureller und nicht – struktureller Vorläuferproteine und

3.) die Reifespaltung des Kapsids.

Der erste Schritt dient somit der Abspaltung der Vorstufe der Hüllenproteine und wird (bei Entero – und Rhinoviren) autokatalytisch durch die Proteinase 2A vorgenommen ("cis" – Aktivität). In der Reihenfolge der Gene liegt die Sequenz der Proteinase 2A (in der Folge 2A genannt) unmittelbar hinter dem für die Hüllenproteine kodierenden Abschnitt. 2A ist somit aufgrund ihrer Lokalisation im Polyprotein die erste nachweisbare enzymatische Funktion des Virus. Die Trennung der Hüllenproteinregion von dem für die Replikation verantwortlichen Abschnitt findet bereits während der Translation des Polyproteins "in statu nascendi" statt. In vitro Experimente zeigten, daß bei Poliovirus diese primäre Spaltung an der P1 – P2 Region intermolekular d.h. "in trans" von der reifen Proteinase 2A vorgenommen werden kann (Kräusslich, H. – G. und Wimmer, E., 1988, loc. cit.). Das Spaltsignal, welches dabei von der Proteinase 2A erkannt wird, wurde einerseits durch direkte Aminosäuresequenzanalyse des N – Terminus von 2A und/oder des C – Terminus von VP1 bestimmt oder andererseits durch Vergleich der Primärstruktur aufgrund von Homolo – giestudien abgeleitet. Es ist dies bei Polio (Pallansch, M.A. et al., 1984, J. Virol., 49, 873 – 880), BEV und HRV14 (Callahan, P.L. et al., 1985, loc. cit.) ein Tyr/Gly –, bei HRV2 (Kowalski, H. et al., 1987, J. Gen. Virol. 86, 3197 – 3200; Sommergruber, W. et al., 1989, loc. cit.) ein Ala/Gly –, bei HRV1B (Hughes, P.J. et al., 1988, J.Gen. Virol. 69, 49 – 58) und HRV89 (Düchler, M. et al., 1987, loc. cit.) ein Val/Gly –, sowie bei Cox B1 (Iizuka, N. et al., 1987, Virology 156, 64 – 73), Cox B3 (Lindberg, A.M. et al., 1987, Virology 156, 50 – 63) und Cox B4 (Jenkins, O. et al., 1987, J. Gen. Virol. 68, 1835 – 1848) ein Thr/Gly – Aminosäurepaar. Dieser Schritt ist für den weiteren Ablauf der viralen Infektion essentiell (Kompartimentierung von Replikation und Virus – assembly). Bei Cardio – und Aphtoviren wird, im Gegensatz zu den Polioviren, diese Spaltung von der Proteinase 3C katalysiert (Kräusslich, H. – G. and Wimmer, E., 1988, loc. cit.). Vom Poliovirussystem weiß man, daß wahrscheinlich alle an dieser Reifungsspaltung beteiligten Enzyme viral kodiert sind (Toyoda, H. et al., 1986, Cell 45, 761 – 770). Im Poliovirus findet man drei Typen von Spaltsignalen; bestimmte Q – G – Aminosäurepaare, welche von der viralen Proteinase 3C (im folgenden 3C genannt) erkannt werden, das oben erwähnte Y – G Paar, welches als 2A – Erkennungssignal dient und das bei der Reifespaltung des Kapsids verwendete N – S Spaltsignal.

Wie bereits dargelegt wurde, ist der Infektionsablauf bei Picornaviren entscheidend von den viralen Enzymen abhängig. Da gerade diese Enzyme besonders gut konserviert und in ihren Eigenschaften bei verschiedenen Rhinoviren sehr ähnlich sind, bieten sie sich geradezu als Ziel eines chemotherapeutischen Eingriffs an, wie z. B. das virale Enzym 2A. Der chemotherapeutische Ansatz ist die Inhibierung der enzymatischen Aktivität durch spezifische Inhibitoren. Inhibiert man die erste proteolytische Aktivität, nämlich 2A, so unterbindet man jeden weiteren Reifungsprozeß des viralen Systems. Überraschenderweise weist die 2A – Region von HRV2 nicht nur zu anderen Rhinoviren, sondern auch zu Vertretern anderer Gruppen der Picornaviridae eine ausgeprägte Homologie auf. Ein Inhibitor gegen HRV2 2A könnte daher auch auf andere Picornaviren anwendbar sein.

Die generelle Bedeutung der Inhibierung von viral kodierten Proteinasen wurde nicht zuletzt durch Arbeiten mit der Proteinase des humanen Immundefizienz Virus 1 (HIV I) wieder in den Blickpunkt von möglichen antiviralen Therapieansätzen gerückt. Durch Deletions − und Punktmutationen in der Proteina − seregion dieser Art von Retroviren konnte die essentielle Rolle der Proteinase bei der Reifung dieser Virenklasse erkannt werden (Katoh, I. et al., 1985, Virol. 145, 280 − 292; Kohl, N. E. et al., 1988, Proc. Natl. Acad. Sci. USA, 85, 4686 − 4690; Crowford, S. und Goff, S. P., 1985, J. Virol. 53, 899 − 907). Durch Röntgenstrukturanalysen und molekularbiologische Studien konnte weiterhin gezeigt werden, daß die Proteinase von HIV I dem Asp − Typ angehört, sich selbst am Vorläuferprotein prozessieren kann (auch in rekombinanten prokaryontischen Systemen), "in trans" spezifisch Peptide spalten kann und als aktive Proteinase in einer homodimeren Form vorliegt (Navia, M. A. et al., 1989, loc. cit.; Meek, T.D. et al., 1989, loc. cit.; Katoh, I. et al., 1985, loc. cit.). Aufgrund der Tatsache, daß die Proteinase von HIV I in ihrer aktiven Form als Dimer vorliegt, wurde von Wlodawer und Mitarbeitern auch die Entwicklung spezifischer Dimerisierungs − Inhibitoren vorgeschlagen (Wlodawer, A. et al., 1989, Science 245, 616 − 621). Die Ent − wicklung von hochspezifischen kompetitiven Inhibitoren gegen die Proteinase von HIV I auf der Basis von modifizierten Peptidsubstraten wurde erst kürzlich von Tomasselli und Mitarbeitern beschrieben (Tomasselli, A.G. et al., 1990, Biochem. 29, 264 − 269). Von einem fungiziden Antibiotikum, dem Cerulenin, war schon länger bekannt, daß es eine antiretrovirale Aktivität gegen Rous Sarcoma Virus und Murine Leukemia Virus besitzt (Goldfine, H. et al., 1978, Biochem. Biophys. Acta. 512, 229 − 240; Katoh, I. et al., 1986, Virus Res. 5, 265 − 276; ). Im Fall des HIV I konnte die inhibitorische Wirkung von Cerulenin eindeutig mit der Inhibierung beim proteolytischen Prozessieren des Polyproteins von HIV I in Zusammenhang gebracht werden (Pal, R. et al., 1988, Proc. Natl. Acad. Sci. 85, 9283 − 9286). Ausgehend von dieser Tatsache gelang Blumenstein und Mitarbeitern die Entwicklung von spezifischen Inhibitoren gegen die Proteinase von HIV I auf der Basis von synthetischen Nicht − Peptid Inhibitoren. Sie konnten nämlich die inhibitorische Wirkung des Cerulenin auf die Wechselwirkung des elektrophilen Epoxidrestes mit nukleophilen Regionen der Proteinase zurück − führen. Außerdem konnte durch die Entwicklung von synthetischen Derivaten die ursprüngliche Toxizität des Cerulenin vermindert werden (Blumenstein, J.J. et al., 1989, Biochem. Biophys. Res. Commun. 163, 980 − 987)

Auch im picornaviralen System sind derzeit verschiedenste anorganische und organische Verbindungen, sowie Peptidderivate und Proteine bekannt, die eine inhibitorische Wirkung auf das proteolytische Prozes − sieren dieser Viren besitzen. Der Effekt dieser Substanzen beruht auf der direkten Wechselwirkung mit den Proteinasen (Kettner, C. A. et al., 1987, US Patent Nr.: 4,652,552; Korant, B. D. et al., 1986, J. Cell. Biochem. 32, 91 − 95) und/oder auf dem indirekten Weg der Wechselwirkung mit Substraten dieser Proteinasen (Geist, F. C. et al., 1987, Antimicrob. Agents Chemother. 31, 622 − 624; Perrin, D. D. und Stünzi, H., 1984, Viral Chemotherapy 1, 288 − 189). Das Problem bei den meisten dieser Substanzen ist die relativ hohe Konzentration, die zur Inhibierung nötig ist und die zum Teil große Toxizität dieser Verbindun − gen. Die bereits erfolgreiche Anwendung von modifizierten Peptiden und Peptidomimetica als Therapeutika in nicht − viralen Gebieten (Fauchere, J. L., 1986, Advanc. Drug Res. 15, 29 − 69) und das "inhibitor designing" ausgehend von bekannten Strukturen (DesJarlais, R.L. et al., 1989, "Viral Proteinases as Targets for Chemotherapy", in Curr. Commun. Mol. Biol., Cold Spring Harbor Laboratory Press, 203 − 210) sowie die Zunahme an molekularbiologischen und physikalischen Daten über picornavirale Proteasen erweitert das Verständnis von Struktur und Funktion dieser viralen Enzyme und ermöglicht so ein schrittweises rationales "designing" − ausgehend von modifizierten Peptidsubstraten − bis hin zu hochspezifischen und nicht toxischen Peptidomimetica.

Der erste Nachweis einer zweiten viral kodierten Proteinase, nämlich 2A, gelang mittels Antikörpern, die gegen Polio − 3C entwickelt wurden und eindeutig sämtliche an Q − G durchgeführten Spaltungen, nicht aber die Spaltungen zwischen Y − G unterbanden (Hanecak, R. et al., 1982, Proc. Natl. Acad. Sci. USA 79, 3973 − 3977).

Diese Beobachtung führte zum Schluß, daß das proteolytische Prozessieren an Y − G Stellen einer eigenen Proteinase bedarf. Der Sitz dieser zweiten proteolytischen Aktivität konnte in Poliovirus eindeutig 2A zugeordnet werden. Interessant war der Befund, daß 2A eine alternative Spaltung in der Proteinase − Polymeraseregion (3CD) durchführt, welche ebenfalls an einer Y − G Stelle stattfindet und inaktive Enzyme 3C' und 3D' liefert. Diese Spaltung dient möglicherweise zur quantitativen Regulation der Enzyme 3C und 3D (Lee, C.K. und Wimmer, E., 1988, Virology 166, 1435 − 1441).

Da es während der Infektion mit Poliovirus in HeLa − Zellen sehr rasch zu einem Abschalten der Wirtspro − teinsynthese kommt, die Translation der Poliovirus − RNA jedoch unbehindert ablaufen kann, nahm man an, daß ein oder mehrere Regulationsfaktoren der Translation während der Infektion verändert werden. Tat − sächlich zeigen ältere Befunde, daß der eukaryontische Initiationsfaktor 4F durch proteolytische Spaltung der p220 Komponente während der Poliovirusinfektion in HeLa − Zellen verändert wird (Etchison, D. et al.,

1984, J. Virol. 51, 832 – 837; Etchison, D. et al., 1982, J. Biol. Chem. 257, 14806 – 14810; Etchison, D. und Etchison, J.R., 1987, J. Virol. 61, 2702 – 2710). In der Folge konnte gezeigt werden, daß 2A indirekt für diese Modifikation von p220 in infizierten Zellen verantwortlich ist (Kräusslich, H. G. et al., 1987, J. Virology 61, 2711 – 2718; Lloyd, R. E. et al., 1986, Virology 150, 299 – 303; Lloyd, R. E. et al., 1987, J. Virol. 61, 2480 – 2488).

Die Frage nach der Transaktivität der Proteinase 2A konnte zunächst im Poliovirussystem positiv beant – wortet werden, indem durch Insertionen und Deletionen in 2A eine unprozessierte P1 – P2 Region in E. coli exprimiert wurde, die von 2A gespalten werden konnte, die entweder aus mit Poliovirus infizierten Zellen stammte (Nicklin, M. J. H. et al., 1987, Proc. Natl. Acad. Sci. USA 84, 4002 – 4006) oder "in vitro" translatiert wurde (Kräusslich, H. – G. et al., 1987, J. Virol. 61, 2711 – 2718) oder in E. coli exprimiert wurde, oder aus Poliovirus infizierten Zellen gereinigt wurde (König, R. und Rosenwirth, B., 1988, J. Virol. 62, 1243 – 1250). Bernstein und Mitarbeiter konnten mit Hilfe einer Poliovirusmutante klar zwischen einer "cis" – und einer "trans" – aktiven Form der Proteinase 2A unterscheiden. Diese 2A – Mutante enthielt ein zusätzliches Leu zwischen der 102. und 103. Aminosäure von 2A. Diese Leu – Insertion führte zwar zu einem schlecht replizierenden, aber dennoch lebensfähigen Poliovirus. In mit dieser Mutante infizierten HeLa – Zellen konnte dagegen keine Spaltung des zellulären p220 Moleküls beobachtet werden (Bernstein, H.D. et al., 1985, Mol. Cell. Biol. 5, 2913 – 2923; Bernstein, H. D. et al., 1986, J. Virol. 60, 1040 – 1049).

Mit Hilfe von rekombinanten Vaccinia Vektoren, die die vollständige P1 – Region und den Genabschnitt für eine verkürzte inaktive Form der Proteinase 2A aus Polio enthielten, konnte gezeigt werden, daß nach Koinfektion mit jeweils einem der drei Serotypen von Polio, mit HRV14 oder mit EMCV sowohl in den Fällen von Polio 1,2 und 3 als auch von HRV14 ein korrektes intermolekulares ("trans") Prozessieren an der P1/2A Schnittstelle erfolgte. Nach Koinfektion mit EMCV fand kein Prozessieren des inaktiven Vorläuferproteins von Polio statt (Jewell, J. E. et al., 1990, J. Virol. 64, 1388 – 1393). Ferner konnte in höheren eukaryonti – schen Zellen mit Hilfe von Expressionsvektoren, welche unter der Kontrolle der LTR – Region von HIV I stehen und mittels des "tat" – Genprodukts aktiviert werden, der Beweis erbracht werden, daß 2A von Poliovirus das induzierende Agens bei der Proteolyse von p220 ist (Sun, X. H. und Baltimore, D., 1989, Proc. Natl. Acad. Sci. 86, 2143 – 2146). Weiterhin zeigten Kräusslich und Mitarbeiter, daß zwar die Anwesenheit einer aktiven 2A zur proteolytischen Degradation von p220 während des "host cell shut off" unbedingt notwendig ist, dieser Abbau aber nicht direkt von 2A durchgeführt wird (Kräusslich, H. – G. et al., 1987, loc. cit.). Man vermutet zur Zeit, daß die Proteinase 2A in der Lage ist, ein zelluläres Enzym zu aktivieren, welches in der Folge die Spaltung von p220 bewirkt. Neuere Untersuchungen geben Anlaß zu Spekulationen, daß möglicherweise die $Ca^{2+}$ – abhängige zelluläre Proteinase Calpain für diese "p220 – ase" Aktivität in Betracht zu ziehen ist. Nach diesem Modell soll die Proteinase 2A ein inaktives Calpain durch dessen Spaltung in eine aktive Form überführen, die in der Folge das p220 attackiert. Die mögliche Spaltung des Calpains könnte dabei 80 Aminosäuren vom N – Terminus entfernt bei der Sequenz Y – G stattfinden (Wyckoff, E. E. und Ehrenfeld, E., 1989, EUROPIC 89, "Sixth Meeting of the European Study Group on the Molecular Biology of Picornaviruses", Bruges, Belgium).

Möglicherweise sind die beiden Proteinasen 2A und 3C direkt oder indirekt an der proteolytischen Degradation von weiteren 14 zellulären Proteinen während der Poliovirusinfektion von HeLa – Zellen beteiligt (Urzainqui, A. und Carrasco, L., 1989, J. Virol. 63, 4729 – 4735).

Neueste Daten zeigen, daß Poliovirus Proteinase 2A nicht nur die Translation der Wirtszelle, sondern auch die DNA – Replikation und die RNA Polymerase II Transkription inhibiert (Davies, M. V. et al., 1991, J. Biol. Chem 266, 14714 – 14720).

Diesen Daten zufolge, müßten die verschiedenen 2A Proteinasen der einzelnen Entero – und Rhinovi – ren ein oder mehrere gemeinsame "targets" in der Zelle erkennen. Deshalb ist das Verstandnis und das Wissen über die Trans – Substratspezifität der 2A von großer Bedeutung.

Die Expression der 2A von HRV2 ist bislang nur als Fusionsprotein möglich. Durch die autokatalytische Aktivität spaltet sich nach Expression die reife Proteinase aus dem Vorläufermolekül ab. Wegen der geringen Löslichkeit des Vorläuferproteins und der abgespaltenen Proteinase selbst, ist dieses Verfahren jedoch mit einem großen Ausbeuteverlust verbunden, so daß die freigesetzte reife Proteinase in einer sehr geringen Konzentration vorliegt (Sommergruber et al., 1989, loc. cit.).

Es ist die Aufgabe der vorliegenden Erfindung, ein Verfahren zur Expression einer reifen HRV2 2A Proteinase, ein einfaches Verfahren zur partiellen Reinigung, Peptidsubstrate zur Aufklärung der Trans – Substratspezifität sowie inhibitorisch wirkende Peptide bereitzustellen.

Überraschenderweise wurde nun ein Verfahren gefunden, das die bakterielle Expression der reifen Proteinase 2A sowohl mit guter Ausbeute als auch mit relativ großer Löslichkeit erlaubt. Dazu wird die für die Proteinase kodierende DNA (Sommergruber et al., 1989, loc. cit.) in einen induzierbaren Vektor kloniert. Beispiel 1 beschreibt in einer vorteilhaften Ausgestaltung der Erfindung die Klonierung des BstEII/HindIII –

5

Fragmentes aus pEx2A (Sommergruber et al., 1989, loc. cit.), das den größten Teil der kodierenden Region der 2A Proteinase mit Ausnahme der ersten 11 N−terminalen Aminosäuren umfaßt, sowie des Oligonu− kleotids

```
5−        CATGGGCCCGAGTGACATGTATGTTCATGTAG             −3
3−              CCGGGCTCACTGTACATACAAGTACATCCAATG      −5,
```

das die fehlenden N−terminalen 2A−Aminosäuren einschließlich eines Startcodons umfaßt, in den mit NcoI und HindIII linearisierten Expressionsvektor pROK−1 (CLONTECH Laboratories). Eine analoge Vorgangsweise ist auch für die Expression enzymatisch nicht aktiver, reifer HRV 2A−Varianten möglich. Beispiel 1 beschreibt die Klonierung entsprechender BstEII/HindIII−Fragmente aus pEx2A (Cys106:Ser) und pEx2A (ΔGly/107/108) (Sommergruber et al., 1989, loc. cit.). Zur Expression der 2A wird der das komplette Gen enthaltende Vektor, vorteilhafterweise pROK−1/2A−41 oder für eine enzymatisch nicht aktive Variante pROK−1/9B oder pROK−1/I1 in E. coli, bevorzugt in E. coli HB 101, nach bekannten Methoden transformiert. Die Anzucht kann dann z. B. in LB−Medium bei 35˚C (unter Zusatz des entsprechenden Antibiotikums) für 8 Stunden erfolgen ($OD_{600}$ = 1,75) und nach Verdünnung (1:10) und weiterer Inkubation für 2 Stunden ($OD_{600}$ = 1,10) mit IPTG (Endkonzentration : 0,5 mM) induziert werden. Nach ca. 13 stündiger Induktion ($OD_{600}$ = 2,2) können die Zellen aufgearbeitet werden. Überraschenderweise liegt unter den beschriebenen Anzuchtbedingungen ein Maximum der Proteinase in löslicher Form vor (ca. 15 bis 20 % der gesamten 2A−Menge).

Die durch Zentrifugation abtrennbaren Zellen können mit an sich bekannten Methoden, z.B. Ultraschall, aufgebrochen werden. Der aus dem Zellextrakt durch Zentrifugation (Beispiel 2) erhaltene Überstand kann durch Ionenaustauschchromatographie, bevorzugt an MONO Q−Material, weiter gereinigt werden. Überraschenderweise gelingt durch diesen einzigen Aufreinigungsschnitt die Abtrennung der interferierenden proteolytischen Aktivität, die durch die endogenen E. coli−Proteinasen bedingt ist (Beispiel 3). In einer vorteilhaften Ausgestaltung wird der nach Zentrifugation und Ultrazentrifugation gereinigte Überstand (Puffer: 50 mM Tris−HCl (pH 8,5), 5 mM DTT) auf eine MONO Q 5/5 Säule gegeben. Eluiert wird mit einem Salzgradienten, vorzugsweise mit einem 30 % bis 50 % NaCl−Gradienten (Elutionspuffer A: 50 mM Tris− HCl (pH 8,5), 5 mM DTT; Elutionspuffer B: 50 mM Tris−HCl (pH 8,5), 5 mM DTT, 1 M NaCl). Die Fraktionen, die die 2A enthalten, können z.B. durch die korrekte Spaltung des 16−mer Peptids P89, das der Wildtyp−Schnittstelle entspricht, erkannt werden (Beispiel 3, Sommergruber et al. 1989, loc. cit.). Zur Identifizierung der eine inaktive Proteinase enthaltenen Fraktionen können an sich bekannte immunologische Methoden verwendet werden (Beispiel 2 und 3, Sommergruber et al. 1989, loc. cit.). Die Fraktionen, die die Proteinase 2A enthalten, werden vereinigt, auf 8 % Glycerin, 1 mg/ml acetyliertes BSA (Rinderserumalbumin) gebracht und können nach Einfrieren in flüssigem Stickstoff bei −20˚C gelagert werden. Die enzymatische Aktivität bleibt bei einem geringen Verlust von 5 bis 10 % größtenteils erhalten.

Nach diesem Verfahren können alle HRV 2A Proteinasen, bevorzugt die HRV2 2A Proteinase sowie die durch Mutationen, Deletionen und/oder Insertionen herstellbaren aktiven und inaktiven HRV2 2A−Proteinasevarianten, bevorzugt die inaktiven Varianten 9B und 1I, erhalten werden. Der korrekte N−Terminus kann z.B. durch Aminosäuresequenzierung überprüft werden. Da ein großer Teil des nach dem beschriebenen Verfahren hergestellten Proteinmaterials für eine Ansequenzierung blockiert ist, enthält jede 2A Präparation bevorzugt formyliertes N−terminales Methionin.

Damit umfaßt die Erfindung DNA−Vektoren, die eine für eine HRV 2A Proteinase kodierende Sequenz enthalten, sowie die durch Mutationen, Deletionen und/oder Insertionen herstellbaren Varianten, die für eine enzymatisch aktive oder nicht aktive HRV 2A Proteinase kodieren und dadurch gekennzeichnet sind, daß sie Expression des reifen Proteins erlauben. Bei den DNA−Vektoren kann es sich dabei bevorzugt um die Plasmide pROK−1/41, pROK−1/I1 oder pROK−1/9B handeln. Weiterhin umfaßt die Erfindung Wirtsorganismen, die die oben beschriebenen Vektoren enthalten, bevorzugt E. coli, besonders bevorzugt E. coli HB 101. Ebenfalls umfaßt die Erfindung ein Verfahren zur Expression der oben genannten Proteinasen, dadurch gekennzeichnet, daß ein Wirtsorganismus mit einem oben genannten DNA−Vektor transformiert, die entsprechende Proteinase zur Expression gebracht und gereinigt wird sowie ein Verfahren zur Reinigung einer rekombinant hergestellten Proteinase, dadurch gekennzeichnet, daß die entsprechende Proteinase wie oben beschrieben zur Expression gebracht und mittels anionischer Ionenaustauschchromatographie, bevorzugt mittels Mono Q−Material, gereinigt wird.

Mit der Bereitstellung einer enzymatisch aktiven Proteinase bzw. einer teilgereinigten Fraktion einer enzymatisch aktiven Proteinase ist nun die Möglichkeit eröffnet, die Einwirkung der Proteinase auf Substrate zu untersuchen.

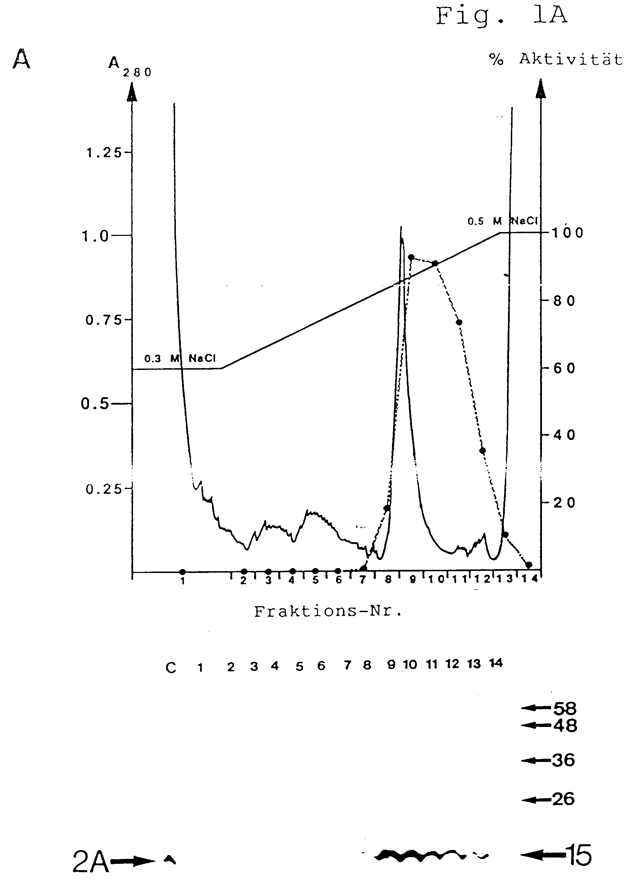

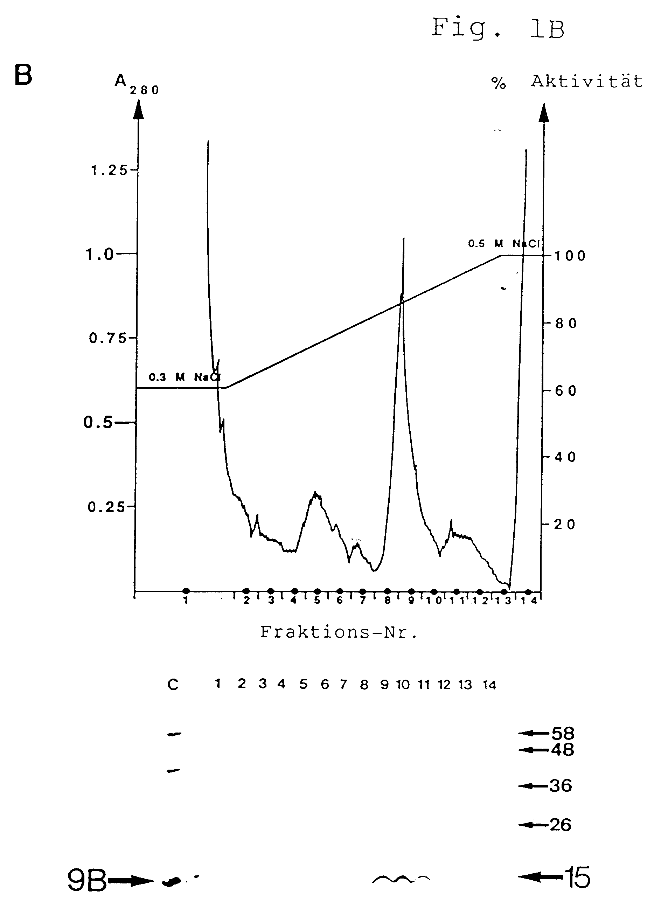

Zur Analyse der Spaltungskinetik von Substraten der 2A Proteinase kann das Verfahren aus Beispiel 3 angewendet werden. Die mittels Chromatographie an MONO Q – Säulen partiell gereinigten Enzympräpa – rationen werden mit einem Peptidsubstrat versetzt und die Produkte mit Hilfe der "reverse – phase" – HPLC detektiert und/oder durch die Komigration von Vergleichspeptiden und/oder durch Aminosäureanalyse identifiziert. Bei dem Peptidsubstrat kann es sich z.B. um das 16 – mer – Peptid "P89" (acetyl – TRPIITTA*GPSDMYVH – NH$_2$) handeln, das die Wildtyp – Schnittstelle der Proteinase 2A enthält und spe – zifisch am Aminosäurepaar Ala – Gly gespalten wird (Fig. 1A).

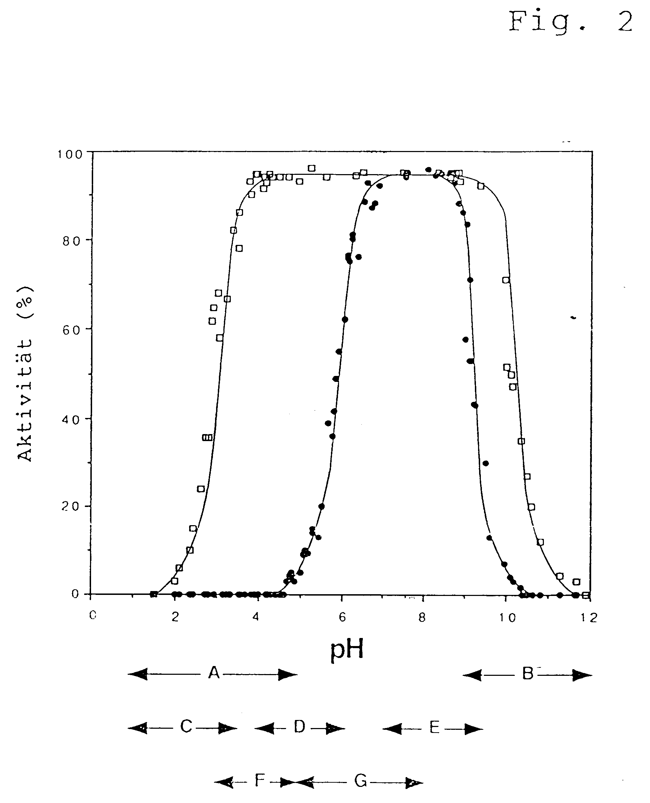

Zur Analyse der Peptidspaltung ("Trans – Spaltungs – Assay") werden 100 $\mu$l einer MONO Q – Fraktion, die eine 2A – Proteinase enthält, mit dem entsprechenden Peptid (5 $\mu$l einer wäßrigen Peptidlösung mit 4 bis 2 mg/ml) bei 34°C für 2, 4, 8, 16 und 32 Minuten inkubiert. Für Kompetitions – oder Inhibitorexperi – mente wird die entsprechende Substanz in 5 $\mu$l Volumen zugegeben. Die Reaktionen werden mit 0,5 M HClO$_4$ gestoppt. Nach weiterer Aufarbeitung (Beispiel 3) können die Reaktionsprodukte wie beschrieben analysiert werden. Die auf diese Weise durchgeführte Analyse der pH – Abhängigkeit der HRV2 2A Proteinase in Beispiel 3 zeigt Figur 2. Aus der Abbildung geht hervor, daß die Proteinase 2A bei kleineren pH – Werten nicht irreversibel zerstört wird. Das Enzym erhält seine Aktivität nach Retitration von pH Werten zwischen 4 bis 9 auf ca. pH 8,5 zurück. Die Kurve zeigt demnach, daß kinetisch wichtige Ionisierungen eher an sauren als an basischen Aminosäureseitengruppen auftreten.

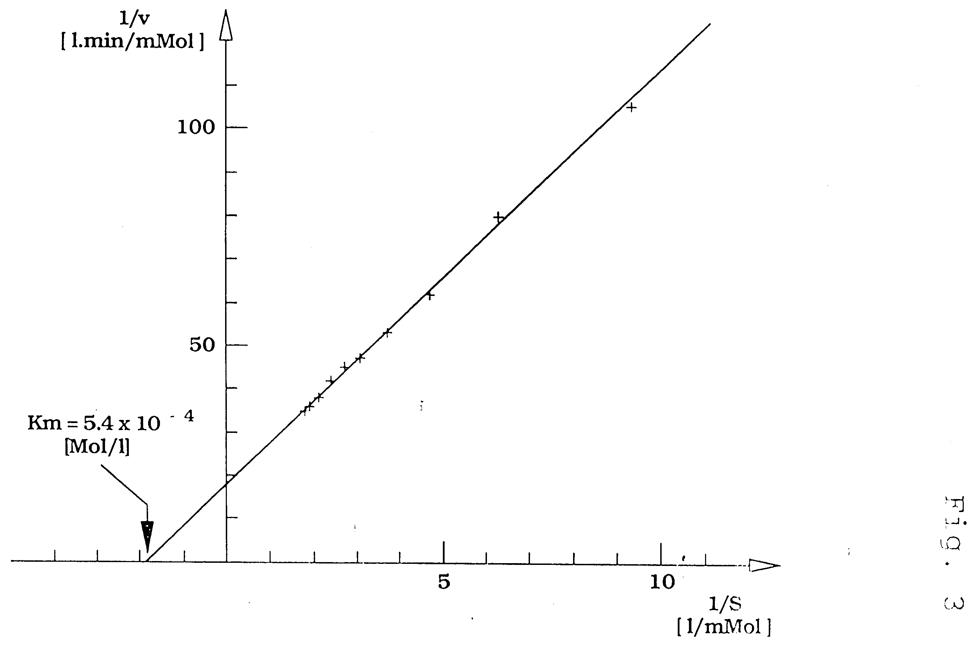

Trans – Spaltungs – Assays wurden auch zur Km – Wertbestimmung ((5,4 ± 0,02) x 10$^{-4}$ mol/l) mit dem Peptidsubstrat P89 durchgeführt (Beispiel 5, Fig. 3).

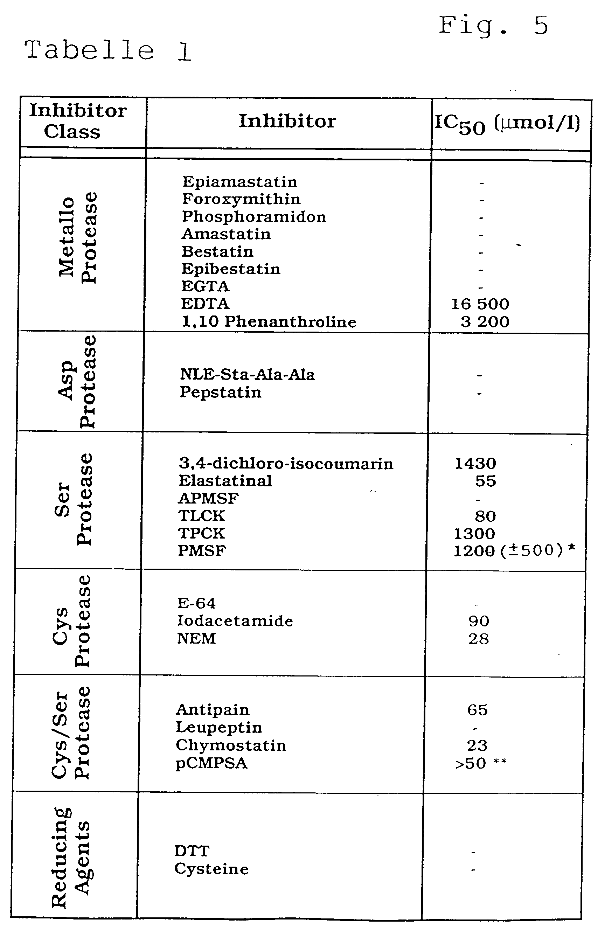

Der Effekt einer Reihe bekannter Inhibitoren von Serin –, Cystein –, Aspartat – und Metalloproteinasen als auch der Effekt reduzierender Agentien auf die Aktivität der 2A gegenüber dem Peptid P89 kann mit Hilfe von Trans – Spaltungs – Assays untersucht werden. Die Trans – Spaltungs – Assays wurden wie oben beschrieben durchgeführt, nur daß die verschiedenen Proteinaseinhibitoren vor Reaktionsbeginn zugegeben wurden. Die kinetischen Untersuchungen, die mit frisch präparierten und stabilisierten 2A – Präparationen dreimal wiederholt wurden, zeigen einen hohen Grad an Reproduzierbarkeit. Tabelle 1 (Fig. 5) zeigt, daß spezifische Aspartat – und typische Metalloproteinaseinhibitoren keinen Einfluß auf die Transaktivität haben, mit Ausnahme von EDTA und 1,10 – Phenantrolin, die einen gewissen inhibierenden Effekt bei höheren Konzentrationen zeigen. Ein ähnlicher Effekt ist für einige Trypsin – ähnliche Proteinasen und für Calpaine beschrieben worden, die zwar durch Ca$^{2+}$ stabilisiert werden, aber nicht davon abhängig sind. (North, 1989, in: "Prevention of unwanted proteolysis" in Proteolytic Enzymes (a practical approach), Hrsg.: Beyon, R. J. und Bond, J. S., IRL – Press Oxford). SH – reaktive Agentien wie Iodacetamid und N – Ethylmaleimid inhibieren die Proteinase 2A stark, während E – 64 (ein spezifischer Cys – Proteinase Inhibitor) keinen Effekt zeigt. Ein Peptidaldehyd – Inhibitor der Cys – Proteinasen (Antipain) und zwei Peptidaldehyd – Inhibitoren der Ser – Proteinasen (Chymostatin und Elastatinal) sind sehr effektiv gegen 2A. Die Wirkungslosigkeit von Leupeptin (ein Peptidaldehyd – Inhibitor der Cys – Proteinasen) könnte auf die geringe effektive Konzentra – tion zurückgeführt werden, die möglicherweise durch Hydratisierung der Aldehydgruppe bewirkt wird (Beynon und Salvesen, 1989, in: "Proteolytic enzymes a paractical approach"; S. 241 – 249, Hrsg.: R. J. Beynon und J. S. Bond, IRL Press, Oxford). TLCKL, PMSF und TPCK, die bekannt sind für ihre inhibitorische Wirkung auf Cysteinproteinasen durch unspezifische Alkylierung von Sulphhydrylgruppen der Enzyme (Rich, 1986 a, in: "Proteinase Inhibitors", S. 153 – 178. Hrsg.: A. J. Barrett und G. Salvesen, Amsterdam, Elsevier), sind nur wirksam bei höheren Konzentrationen. Andere Ser – Proteinase – Inhibitoren, wie APMSF und 3,4 – Dichloroisocumarin (Beynon und Salvesen, 1989, loc. cit.), zeigen keine Aktivität gegen die HRV2 2A. Reduzierende Agenzien wie DTT und Cystein haben keinen Effekt. Auf der Grundlage der Untersuchungen von Bazan und Fletterick (1989, FEBS Lett. 249, 5 – 7) und Sommergruber et al. (1989, loc. cit.) wurde Cystein (Cys 106) als aktives Nucleophil vorgeschlagen. Jedoch zeigen die Untersuchungen mit Polio 2A von Koenig und Rosenwirth, (1988, J. Virol., 62, 1243 – 1250) und Yu und Lloyd, (1991, Virology, 182, 615 – 625), als auch die hier vorliegenden Daten, daß die Proteinasen, hier die HRV2 2A, nicht eindeutig in die Gruppe der Cysteinproteinasen einzuordnen sind.

Im folgenden wird die Bereitstellung inhibitorisch wirkender Substrate der HRV2 2A Protease beschrie – ben, die dadurch gekennzeichnet sind, daß sie von dem Aminosäuremotiv TRPIITTAGPSDMYVH abgeleitet sind und durch C – oder N – terminale Verkürzung erhalten werden und/oder das Aminosäuremotiv ITTAGPS verändert ist.

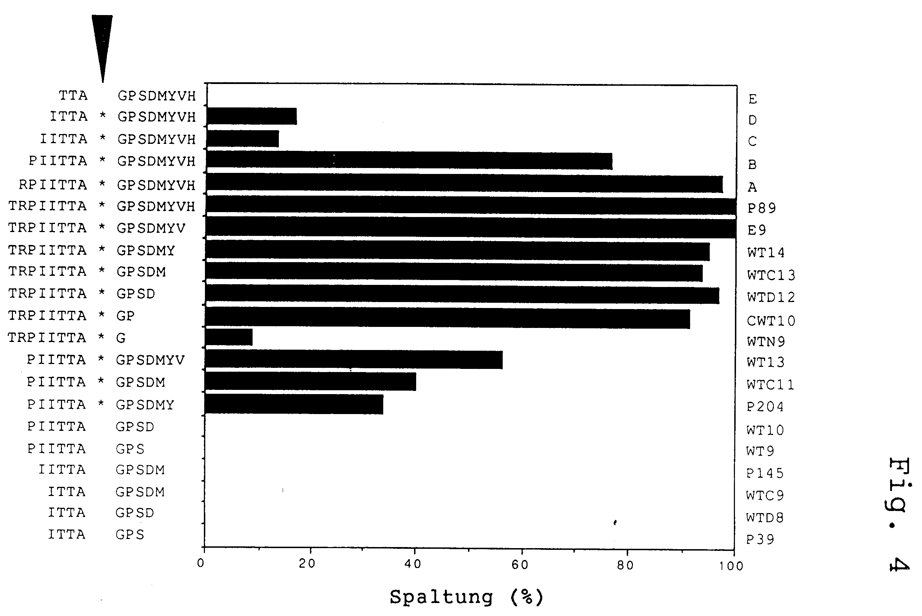

Es wurde gezeigt, daß ein 16 Aminosäuren langes Peptid (P89), das die Region zwischen dem C – Terminus von VP1 und dem Aminoterminus von 2A umfaßt, als effektives Substrat für die Proteinase 2A dienen kann (Sommergruber et al., 1989, loc. cit.). Die Substratfähigkeit kürzerer Peptide wurde mittels Trans – Spaltungs – Assays überprüft (unter identischen Bedingungen wie in Beispiel 3 beschrieben) und mit P89 verglichen. Die Ergebnisse sind in Figur 4 zusammengefaßt. Sukzessive C – terminale Deletionen innerhalb von P89 (Peptide E9, WT14, WTC13, WTD12, CWT10 und WTN9) haben weit weniger Einfluß auf die Spaltbarkeit, als N−terminale Deletionen (Peptide A, B, C, D und E). Längenreduktion des aminoterminalen Endes von P89 über die Aminosäure Prolin (Position P6) führt zu einem Abfall der Spaltbarkeit um eine Größenordnung. Dieser Teil des Peptides ist entscheidend für eine effektive Erkennung und Spaltbarkeit (Peptide C und D). Demgegenüber ist jedoch die Mindestzahl von einer Aminosäure (Peptid WTN9) auf der carboxy−terminalen Seite für eine Spaltung ausreichend. Demzufolge spielt das C−terminale Ende eine kleinere Rolle für die Substraterkennung und/oder Spaltung. Abbau von beiden Seiten des Peptidsubstrates führt zu einem 11−mer Substrat (WTC11), das effizient gespalten werden kann (40 %)` Weiterer Abbau von beiden Seiten erlaubt keine Spaltung mehr. Eine Löslichkeitsänderung als Ursache für Unterschiede in der Spaltbarkeit wird unter den Versuchsbedingungen (Beispiel 8) nicht beobachtet.

Rhinovirale 2A Proteinasen erkennen ein Ala oder Val an Position P1 und ein Gly an Position P1', mit Ausnahme der Proteinase 2A von HRV14, die eine Tyr−Gly Bindung spaltet, ähnlich wie bei den Serotypen des Poliovirus. Coxsackie Viren besitzen eine Thr−Gly−Spaltungssequenz an ihrer VP1−2A Schnittstelle. Die meisten Spaltsignale der Rhino− und Enteroviren besitzen ein Thr an Position P2 und/oder P3 (Kräusslich und Wimmer, 1988, loc. cit.). Nur HRV14 und HRV9 besitzen kein Thr an einer dieser Positionen (Stanway et al., 1983, Nucl. Acids Res. 11, 5629 − 5643; Lecki, G.W., 1988, loc. cit.). Weiterhin besitzen alle bekannten Rhinovirus−Stämme ein Prolin an Position P2' mit der Ausnahme von HRV14.

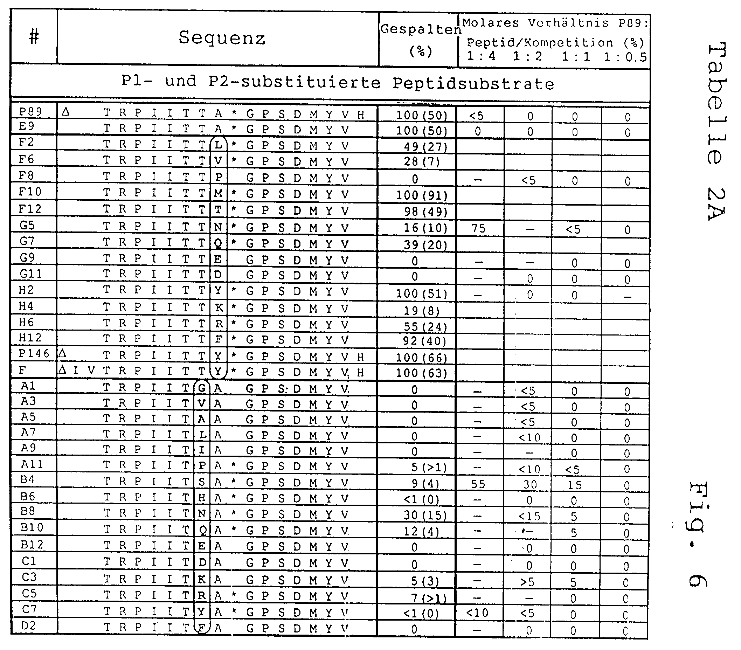

Mit modifizierten Peptidsubstraten (Mutationen an den Positionen P2, P1, P1', P2' und P3' (Beispiel 8)) kann die Spaltungseffizienz bestimmt werden. Als Vergleich kann z.B. ein 15−mer Wildtyp−Substrat (E9, Tabelle 2 (Fig. 6−9)) dienen. Ein Einfluß der Ladungsverteilung kann ausgeschlossen werden (Beispiel 8). Die auftretenden Spaltstellen können durch Komigration mit entsprechenden Referenzpeptiden festgestellt werden.

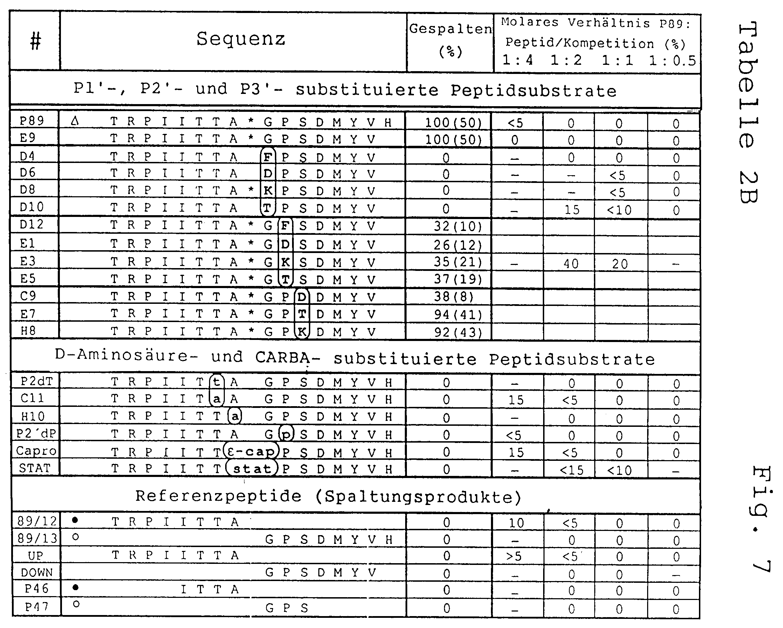

Überraschenderweise stellte sich nun heraus, daß die Mutationsanalysen eine hohe Toleranz gegen Austausche an den Positionen P1, P2' und P3', jedoch eine absolute Abhängigkeit von Threonin an P2 und von Glycin an P1' zeigten (Tabellen 2A und 2B; Fig. 6 und Fig. 7). HRV2 2A toleriert an der Position P2 nur Austausche von Aminosäuren, die an Größe bzw. Polarität dem Thr am nächsten kommen (Peptide B4, B8 und B12; Tabelle 2A). Peptide mit positiv geladenen Resten in Position P2 (Peptide C3 und C5) zeigen eine (wenn auch stark reduzierte) Spaltbarkeit. Eine mögliche Interpretation der Akzeptanz von positiven Ladungen an dieser Position wäre die strukturelle Ähnlichkeit zu Trypsin−ähnlichen Proteinasen (Bazan und Fletterick, loc. cit.), die bekanntermaßen nach basischen Aminosäuren spalten. Alle anderen Mutationen im Peptidsubstrat führen dazu, daß das Peptid nicht mehr von 2A gespalten werden kann (Tabelle 2A). Die Position P2 scheint daher (neben dem ITTA−Motiv und dem Arg in Position P7) eine essentielle Rolle zu spielen. Peptidinhibitoren sollen daher an der Position P2 Reste enthalten, die zu einer starken Wechselwirkung des Substrats mit der Proteinase führen, z.B. die Verwendung von modifizierten Aminosäuren mit reaktiven Seitenketten wie α−Amino−isobuttersäure, Vinylglycin, Homocystein, Homoserin, β−Cyanoalanin oder S−Methylcysteinsulfoxid. Ein Tyr−Gly−Spaltsignal von Poliovirus Stämmen und HRV14 (Kitamura et al., 1981, Nature 291, 547 − 553; Toyoda et al., 1984, J. Mol. Biol., 174, 561−585; Stanway et al. 1983, loc. cit.), als auch Thr−Gly−Signale von den Coxsackieviren B1, B3 und B4 können effizient gespalten werden (Iizuka et al., 1987, Virology, 156, 64−73; Linberg et al., 1987, Virology 156, 50−63; Jenkins et al., 1987, J. Gen. Virol. 68, 1835−1848). Durch Vergleich der %−Spaltbarkeit der Peptidmutanten zu einem Zeitpunkt, bei welchem 50 %−Spaltung der Wildtypsubstrate (P89 und E9) vorliegt, konnte gezeigt werden, daß bei Anwesenheit eines Met oder Tyr in Position P1 (Peptide F10, H2, P146 und F) eine höhere Effizienz an Spaltbarkeit gegeben ist (Tabelle 2A). Diese beiden Reste in P1 dürften für die HRV2 2A eine kinetisch günstigere Trans−Aktivität schaffen.

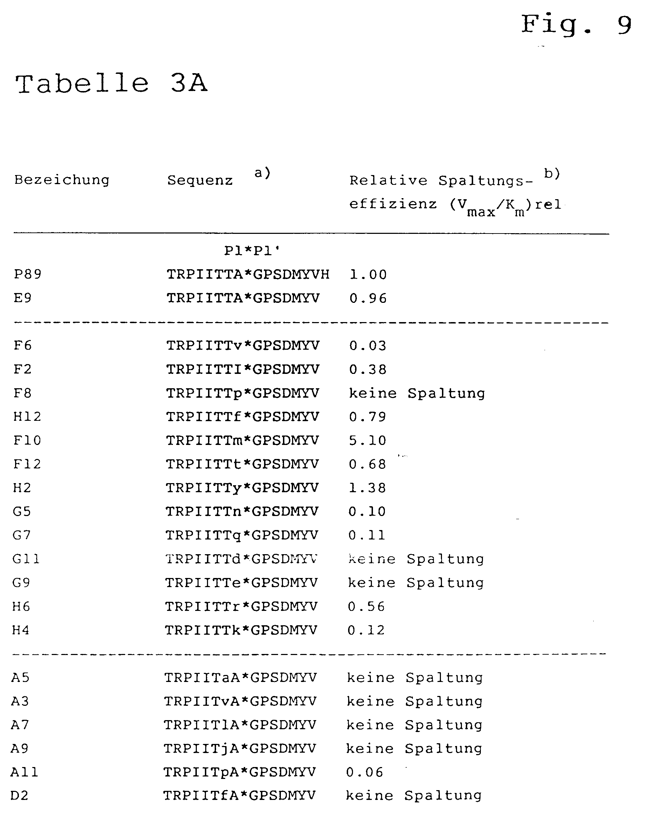

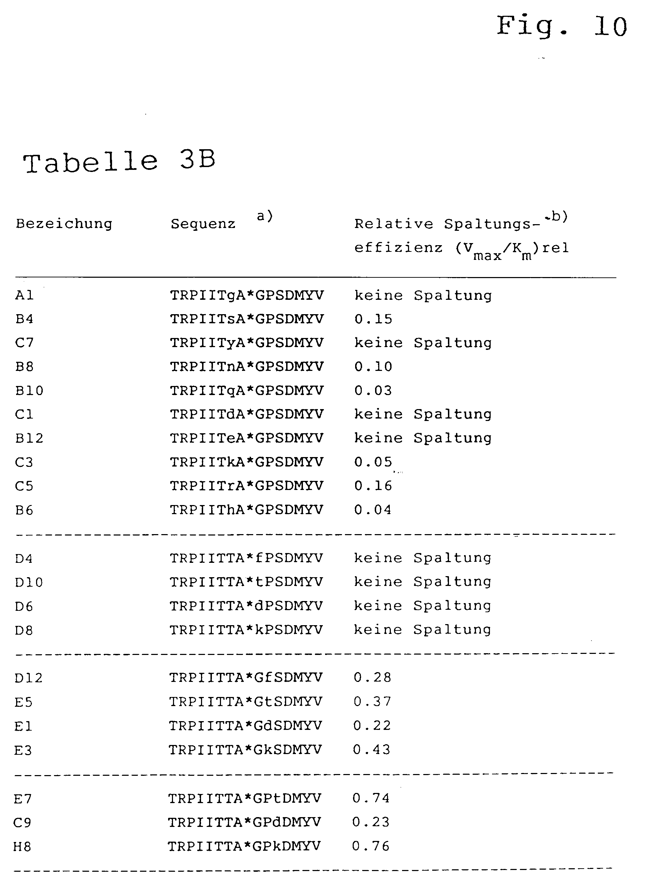

Auch die Bestimmung der relativen Spaltungseffizienz ($V_{max}/K_m)_{rel}$−Werte) nach Pallai et al. (J. Biol. Chem. (1989) 264, 9738−9741) bestätigt die oben gemachten Aussagen. Fig. 9 und 10 zeigen die relative Spaltungseffizienz für Peptide mit einem einzigen Aminosäureaustausch.

Zusammengefaßt zeigen diese Resultate die Bedeutung der Positionen P2 und P1' für die Enzym− Substrat Wechselwirkung und/oder für die Stabilisierung der Peptidstruktur.

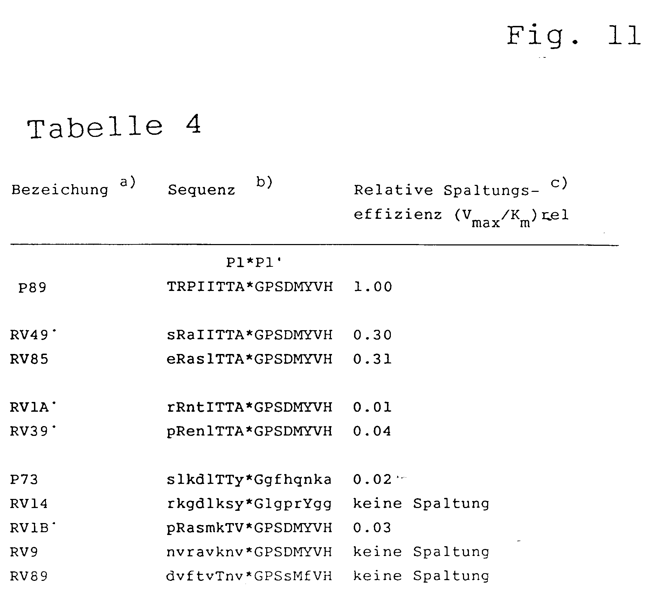

Fig. 11 (Tabelle 4) zeigt die Spaltungseffizienz der HRV2 2A für Peptide, die von anderen rhino− und polioviralen 2A Spaltstellen abgeleitet sind.

Weiterhin können Deletionen in das Wildtyp Substrat eingeführt werden, (Peptide AB1, AB2 und ST2), um zu überprüfen, ob die Proteinase 2A auch andere, weniger dominante Spaltsignale innerhalb eines Peptids, wie sie für die cis−Aktivität von Polio 2A (Hellen et al., 1989, in: "Viral proteinases as targets for chemotherapy", S. 27−32, Hrsg.: Kräusslich, H.−G., Oroszlan, S. und Wimmer, E., Curr. Comm. Mol. Biol., Cold Spring Harbor Laboratory Press) beschrieben wurden, akzeptiert. Keine Spaltung (auch keine alternative Spaltung) wurde für AB1, AB2 und ST2 beobachtet (Tabelle 2C). Die Einführung von D−Aminosäureanaloga bei P2, P1 und P2' (Peptide P2dT, C11, H10 und P2'dP) erlaubt keine Spaltung der modifizierten Substrate, was eindeutig für die stereochemische Spezifität der HRV2 2A spricht (Tabelle 2B). Eine CARBA−Substitution des Spaltsignals durch ε−Aminocapronsäure oder Statin (Peptide Capro und STAT) führen zu einem kompletten Verlust der Spaltbarkeit (Tabelle 2B).

Ausgehend von der Spaltstelle VP1−2A und der alternativen Stelle innerhalb 3D des Polioserotyps 1 (Lee und Wimmer, 1988, Virol., 166, 405−414) können Peptidsubstrate (P73 und P75) und ihre C− terminalen Spaltprodukte (P74 und P76) synthetisiert werden.

Obgleich hier ein extremer Unterschied zwischen der Primärsequenz der Spaltstelle von Polio und Rhino vorliegt, wird die VP1−2A Spaltsequenz von Polio durch die Proteinase 2A in geringem Ausmaß akzeptiert. Die alternative Polio−Region in 3D (Peptid 75) dient nicht als Substrat für die HRV2 2A (Tabelle 2C).

Chimäre Peptidsubstrate bestehend aus einem N−Terminus von HRV1A, HRV1B, HRV39 oder HRV49 (Palmenberg, A., in Molecular Aspects of Picornavirus Infection and Detection) pp. 211−231; Hrsg.: B.L. Semler and E. Ehrenfeld, Washington, D.C.: American Society for Microbiology; Hughes et al., 1988, loc. cit.) und aus einem C−terminalen Bereich der von HRV2 (Skern, et al., loc. cit.) abgeleitet ist (Tabelle 2C; Peptide RV1A, RV1B, RV39 und RV49), zeigen die besondere Bedeutung des ITTA−Motivs und/oder des Arg in Position P7.

Die Peptide RV9, RV14, RV85 und RV89 (Leckie, G.W., 1988, loc. cit.; Stanway et al., 1984, loc. cit.; Callahan, 1985, loc. cit.; Stanway, G. 1990, J. Gen. Virol.; 71, 2483−2501 und Duechler et al., 1987, loc. cit.) repräsentieren die Wildtyp−Spaltstelle der entsprechenden Serotypen. Die Peptide RV9, RV14 und RV89, die den größten Unterschied in der Aminosäuresequenz aufweisen (besitzen weder ein ITTA−Motiv noch ein Arg an Position P7) können überhaupt nicht gespalten werden; die Peptide RV39, 1A und 1B können zu ca. 10 % und die Peptide RV85 und 49 können mit guter Effizienz (ca. 50 %) gespalten werden. Dieses Resultat zeigt abermals, daß der N−terminale Bereich der Peptide (besonders das ITTA−Motiv und/oder das Arg in Position P7) entscheidend für eine Spaltung ist. Dies wurde auch durch die verkürzten Wildtyp−Peptide gezeigt (Fig. 4).

Einige P2−substituierte Peptide konnten mit 2A kopräzipitiert werden, was zeigt, daß diese Peptide fest an die Proteinase 2A, möglicherweise in der Nähe des aktiven Zentrums, gebunden, aber nicht gespalten werden. Damit stellt sich die Frage, ob diese oder andere nicht oder nur schwach spaltbare Peptide mögliche kompetitive Inhibitoren von P89 sind.

Nur diejenigen getesteten Peptide verhielten sich kompetitiv, die entweder von verkürzten oder deletierten Wildtyp−Peptidsubstanzen (P145, WTD8 und AB2; Tabelle 2C) oder von Peptiden mit "moderaten" Aminosäureaustauschen an Position P1 (Ala gegen Asn; Peptid G5) oder P2 (Thr gegen Ser oder Asn; Peptid B4 und B8) abgeleitet waren (Tabelle 2A; Fig. 6).

Basierend auf diesen Kenntnissen und mit dem Wissen, das das ITTA−Motiv bzw. das Arg an Position P7 eine große Rolle bei der Substrat−Wechselwirkung spielt, können die erfindungsgemäßen Peptidinhibitoren anstelle der spaltbaren Ala−Gly Stelle C4− oder C6−Aminocarbonsäuren aufweisen, wie z.B. die δ− Aminolävulinsäure, γ−Aminobuttersäure oder Ornithin. Diese Peptide würden zwar noch als Substrat erkannt, aber aufgrund der fehlenden Peptidbindung nicht mehr gespalten werden können. Basierend auf ihrer zu erwartenden höheren Aufenthaltswahrschein− lichkeit sollten die beschriebenen Peptide als kompetitive Inhibitoren wirksam sein.

Eine weitere Möglichkeit ist das Ersetzen des P1− und P2−Restes durch γ−Amino−β−hydroxybuttersäure. Einige P1− und P2−Peptidmutanten (z.B. Peptide G5, B4 und B8) besitzen ein modifiziertes IITTA− Motiv, das noch eine effiziente Wechselwirkung mit der Proteinase erlaubt, aber zu einer drastisch reduzierten Spaltbarkeit führt und die Aufenthaltswahrscheinlichkeit im aktiven Zentrum des Enzyms erhöht.

Der wahrscheinlich kompetitive Wirkungsmechanismus der erfindungsgemäßen, verkürzten und deletierten Peptide (P145, WTD8 und AB2) beruht möglicherweise ebenfalls darauf, daß diese Peptide zwar noch als Substrat über das ITTA−Motiv erkannt werden, jedoch aufgrund ihrer Kürze (P145 und WTD8) oder der fehlenden Spaltstelle (AB2) nicht gespalten werden können. Dies erhöht offensichtlich ihre Aufenthaltswahrscheinlichkeit im aktiven Zentrum und gibt ihnen daher die Eigenschaft von kompetitiven Inhibitoren.

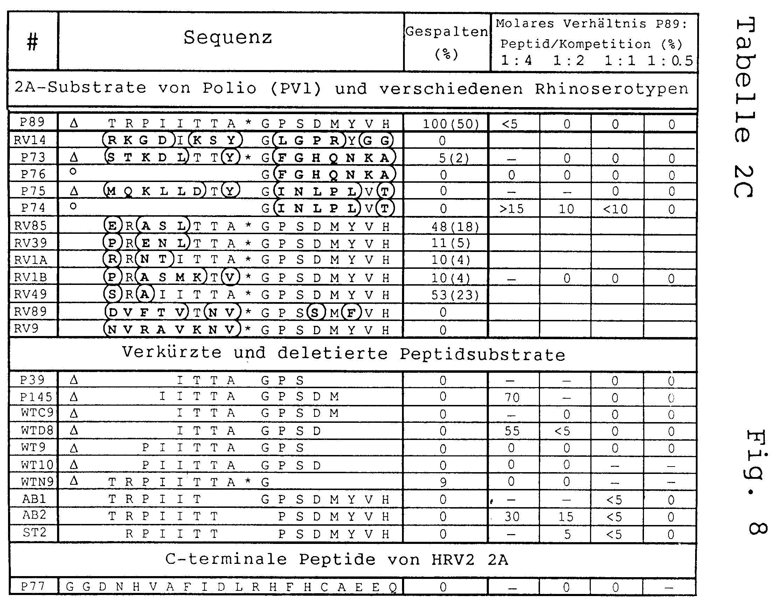

Ein mutiertes Peptid mit einem Aminosäureaustausch Gly gegen Thr an Position P1' konnte nicht gespalten werden, zeigt jedoch eine gewisse Inhibition, die besagt, daß diese Aminosäureposition eine geringe Rolle in der Substraterkennung spielt, aber offensichtlich sterisch bedeutsam für die Spaltung ist. Das gleiche gilt für Peptide, die eine CARBA−Substitution anstelle des HRV2−Spaltsignals Ala−Gly enthalten (Tabelle 2C; Fig. 8; Peptide Capro und STAT).

Zusammengefaßt umfaßt die Erfindung damit außerdem Peptide mit inhibitorischer Wirkung gegen die rhinovirale 2A Proteinase, abgeleitet von dem Aminosäuremotiv TRPIITTAGPSDMYVH, dadurch gekennzeichnet, daß sie N− und/oder C−terminale Verkürzungen enthalten und/oder das ITTAGPS−Teilmotiv verändert ist.

Die Erfindung schließt auch solche Peptide ein, die zusätzliche chemische Gruppen besitzen, die normalerweise nicht Teil dieses Moleküls sind. Diese "chemischen Derivate" enthalten Gruppen, die die Molekülöslichkeit, die Absorption, die biologische Halbwertszeit usw. verbessern oder alternativ die Toxizität oder unerwünschte Nebeneffekte verringern können. Gruppen, die solche Effekte vermitteln, sind bekannt (Remington's Pharmazeutical Sciences (1980)).

Insbesondere umfaßt die Erfindung Peptide und davon abgeleitete chemische Derivate, die wie oben beschrieben von dem Aminosäuremotiv TRPIITTAGPSDMYVH abgeleitet sind wobei eine oder mehrere Aminosäuren deletiert sind und/oder ein moderater Aminosäureaustausch an Position P1 und/oder Position P2 auftritt und oder an Position P2 eine Aminosäure mit einer reaktiven Seitenkette und/oder anstelle der Ala − Gly − Spaltstelle eine C4 − oder eine C6 Aminosäure eingebaut ist und/oder anstelle des P1 und des P2 − Restes $\alpha$ − Amino − $\beta$ − hydroxybuttersäure eingebaut ist.

Bevorzugt sind Peptide mit einer Verkürzung des oben genannten Aminosäuremotives und einer Aminosäuredeletion an P1 und/oder Position P1'. Hierbei kann es sich z.B. um die Peptide P145 (IITTAGPSDM), WTD8 (ITTAGPSD) oder AB2 (TRPIITTPSDMYVH) handeln.

Der Aminosäureaustausch an Position P1 und P2 umfaßt bevorzugt einen moderaten Aminosäureaustausch, wobei ein moderater Aminosäureaustausch für den Austausch einer Aminosäure gegen eine andere mit ähnlicher Größe und Ladungsverteilung steht, z.B. den Ersatz der Aminosäuren des ursprunglichen Aminosäuremotives durch Asn an Position P2. Bevorzugt kann es sich dabei um die Peptide G5TRPIITTNGPSDMYV), B4 (TRIITSAGPSDMYV) und B8 (TRPIITNAGPSDMYV) handeln.

Aminosäuren mit reaktiver Seitenkette können z.B. die Aminosäuren $\alpha$ − Aminobuttersäure, Vinylglycin, Homocystein, Homoserin, $\beta$ − Cyanoalanin, Methylcysteinsulfoxid usw. sein.

Die Ala − Gly − Spaltstelle kann z.B. durch $\alpha$ − Aminolävulinsäure, $\alpha$ − Aminobuttersäure oder Ornithin, $\delta$ − Aminocapronsäure oder Statin ersetzt sein.

Überraschenderweise ermöglicht die Erfindung ebenfalls die Affinität von Peptidensubstraten der HRV2 2A zu verbessern. Insbesondere gilt dies für die Peptide, die an Position P1 Methionin oder Tyrosin anstelle der Aminosäure Alanin des Wildtyps aufweisen. Diese Peptide können dann als Ausgangspunkt für die Bereitstellung von Substraten mit erhöhter Affinität zur HRV2 2A Protease dienen. Die Erfindung umfaßt deshalb auch Peptide mit diesen Substitutionen zur Verbesserung der Substrataffinität.

Die Erfindung umfaßt ebenfalls Arzneimittel zur Inhibierung der HRV2A Proteinase, die die oben beschriebenen Peptide enthalten, gegebenenfalls mit an sich bekannten Hilfs − und/oder Trägerstoffen und/oder Stabilisatoren.

Weiterhin umfaßt die Erfindung die Verwendung der oben beschriebenen Peptide zur Herstellung von Arzneimitteln zur Inhibierung der HRV2A Proteinase sowie ein Mittel enthaltend eines der oben genannten Peptide gegebenenfalls mit an sich bekannten Hilfs − und/oder Trägerstoffen zur Inhibierung der HRV2A Proteinase. Die Erfindung umfaßt weiterhin die Verwendung der oben beschriebenen Peptide zur Inhibierung der HRV2A Proteinase sowie ein Verfahren zur Herstellung der Peptide mittels Fmoc − Strategie mit aktivierten Estern bzw. über HOBt/DIPCDI − Aktivierung sowie nach Analogieverfahren.

Die erfindungsgemäßen Polypeptide sowie ihre pharmakologisch unbedenklichen Säureadditionssalze können in bekannter Weise in die üblichen Formulierungen − wie Tabletten, Dragees, Pillen, Granulate, Aerosole, Siruppe, Emulsionen, Suspensionen und Lösungen unter Verwendung inerter pharmazeutisch geeigneter Trägerstoffe oder Lösungsmittel überführt werden. Hierbei soll der Anteil der pharmazeutisch wirksamen Verbindung(en) jeweils im Bereich von 0,5 bis 90 Gew. − % der Gesamtzusammensetzung liegen, d.h. in Mengen, die ausreichend sind, um den unten angegebenen Dosierungsbereich zu erreichen.

Die Formulierungen werden zum Beispiel durch Verstrecken der Wirkstoffe mit Lösungsmitteln und/oder Trägerstoffen, gegebenenfalls unter Verwendung von Emulgiermitteln und/oder Dispergiermitteln hergestellt, wobei beispielsweise bei der Verwendung von Wasser als Verdünnungsmittel gegebenenfalls organische Lösungsmittel als Lösungsvermittler bzw. Hilfslösungsmittel eingesetzt werden können.

Als Hilfsstoffe seien beispielsweise Wasser, pharmazeutisch unbedenkliche organische Lösungsmittel, wie Paraffine, Öle pflanzlichen Ursprungs, mono − oder polyfunktionelle Alkohole, Trägerstoffe, wie z.B. natürliche Gesteinsmehle, synthetische Gesteinsmehle, Zucker, Emulgiermittel und Gleitmittel erwähnt.

Die Applikation erfolgt in üblicher Weise, vorzugsweise oral oder parenteral insbesondere perlingual oder intravenös. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Die Tabletten können auch aus mehreren Schichten bestehen. Entsprechend können Dragées durch Überziehen von analog den Tabletten hergestellten Kernen mit den üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmackverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethyl − cellulose Netzmittel, beispielsweise Kondensationspro − dukte von Fettalkoholen mit Ethylenoxid oder Schutzstoffe, wie p − Hydroxybenzoate, enthalten.

Injektionslösungen werden in üblicher Weise, z.B. unter Zusatz von Konservierungsmitteln, wie p − Hydroxybenzoaten, oder Stabilisatoren, wie Komplexonen, hergestellt und in Injektionsflaschen oder Am − pullen abgefüllt.

Die Wirkstoffe bzw. Wirkstoffkombinationen enthaltenden Kapseln können beispielsweise in der Weise hergestellt werden, daß man die Wirkstoffe mit inerten Trägern, wie Milchzucker oder Sorbit, mischt und in Gelatinekapseln einkapselt.

Für den Fall der parenteralen Anwendung können Lösungen der Wirkstoffe unter Verwendung geeig − neter flüssiger Trägermaterialien eingesetzt werden.

Die Dosierung für die orale Anwendung liegt bei 1 − 300 mg, vorzugsweise zwischen 5 und 150 mg.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen. Ferner können die erfindungsgemäßen Polypeptide bzw. deren Säureadditionssalze auch mit andersartigen Wirkstoffen kombiniert werden.

## Legenden zu den Abbildungen

**Fig. 1:** $OD_{280}$ − Profile der MONO Q − Fraktionen von aktiver und inaktiver HRV2 2A − Proteinase einschließlich Western Blot − Analyse

Fig. 1A zeigt das Elutionsprofil der Wildtyp Proteinase 2A, Fig. 1B das Elutionsprofil der inaktiven Variante 9B (Cys106:Ser). Der hier verwendete NaCl − Gradient (30 % bis 50 %) ist ebenfalls gezeigt. Die spezifische proteolytische Aktivität (% Spaltung von P89) jeder Fraktion ist durch ausgefüllte Kreise und durch eine unterbrochene Linie angegeben. Der untere Teil der Figuren 1A und 1B zeigt eine Western − Blot − Analyse der MONO Q − Fraktionen. Lage und Molekulargewicht der Markerproteine sind auf der rechten Seite jedes Western Blots angegeben. Große Buchstaben deuten auf die Position der Wildtyp 2A (Fig. 1A) oder auf die Mutante 9B (Fig. 1B) hin. Zahlen am oberen Teil der Western Blots bezeichnen die Fraktionsnummern der MONO Q − Säule. C stellt eine positive Kontrolle dar, die von dem bekannten Expressionssystem für 2A (pEx2A; Sommergruber et al., 1989, loc. cit.) abgeleitet worden ist.

**Fig. 2:** pH − Abhängigkeit der proteolytischen Aktivität von HRV2 2A (% Spaltung von P89 gegen pH)

Geschlossene Kreise stellen die erhaltene Aktivität dar, wenn das Enzym bei einem auf der x − Achse angegebenen pH untersucht wurde. Offene Quadrate stellen die Aktivität dar, die bei Präinkubation von 2A bei dem angegebenen pH − Wert für 20 Minuten bei Raumtemperatur und anschließendem Trans − Spaltungs − Assay der Probe bei pH 8,0 bis 8,5 erhalten wurde.

Pfeile und Großbuchstaben deuten auf das bei diesem Experiment verwendete Puffersystem hin. A: Natriumcitrat/HCl, B: Glycin/NaOH; C: Glycin/HCl; D: Phthalsäure/NaOH; E: Tris/HCl; F: $CH_3COOH$/NaOH und G: $NaH_2PO_4$/$Na_2HPO_4$.

**Fig. 3:** Lineweaver − Burk Diagramm für die $K_m$ − Bestimmung des Peptidsubstrates P89

**Fig. 4:** Mindestlänge von Wildtyp Substraten

Die Sequenzen der Peptidsubstrate, die in dieser Studie verwendet wurden, sind links aufgelistet. Der Stern deutet an, daß das jeweilige Peptidsubstrat an dieser Position gespalten wurde. Der große Pfeil deutet auf die zu erwartete Spaltposition hin. Die Effizienz der Spaltbarkeit jedes Peptides ist in % Spaltung im Vergleich zu P89 angegeben (wie unter den Bedingungen in Beispiel 7 ausgeführt). Die Abkürzungen für die Peptide sind auf der rechten Seite aufgeführt.

**Fig. 5:**  Tabelle 1:

$IC_{50}$ − Werte für HRV2 2A in Gegenwart von bekannten Proteinaseinhibitoren und reduzie − renden Agentien. Ein Stern (*) bedeutet, daß bei Verwendung von PMSF keine lineare Abhängigkeit der Inhibition gezeigt werden konnte (wahrscheinlich ein Löslichkeitspro − blem). Ein Doppelstern (**) bedeutet, daß auf Grund der schlechten Löslichkeit von pCMPSA keine exakte Konzentrationsangabe gemacht werden kann.

**Fig. 6 − 8:**  Tabelle 2:

Spaltbarkeit der Peptidsubstrate und/oder ihre Fähigkeit als Inhibitoren zu dienen.

**Fig. 6:**  Tabelle 2A:

P1 − und P2 − substituierte Peptidsubstrate

**Fig. 7:**  Tabelle 2B: P1' − , P2' − , P3' − ,

D − Aminosäure − und CARBA − substituierte Peptidsubstrate

**Fig. 8:**  Tabelle 2C: Peptidsubstrate abgeleitet von Poliovirus Typ 1 sowie dessen alternative Spaltstelle in 3D (P75) und von Rhinovirus 1A, 1B, 9, 14, 39, 49, 85 oder 89 als auch verkürzte und deletierte Wildtyp Peptidsubstrate.

Eingerahmte, hervorgehobene Buchstaben stellen eine Substitution, Deletion oder Verkür − zung dar. Kleine hervorgehobene Buchstaben beziehen sich auf die D − Aminosäureana − loga. $\epsilon$ − cap und stat bezeichnen den Austausch des Aminosäurepaares Ala − Gly durch $\epsilon$ − Aminocapronsäure bzw. Statin. Ein Stern symbolisiert die identifizierte Spaltstelle.

Die Abkürzungen für jedes Peptid sind auf der linken Seite aufgeführt. Ein offenes Dreieck bezeichnet die Peptide, die eine Acetylgruppe an ihrem Amino − und eine $NH_2$ − Gruppe an ihrem Carboxy − Terminus besitzen. Offene Kreise beziehen sich auf das alleinige Vorliegen einer $NH_2$ − Gruppe am C − Terminus und ein geschlossener Kreis auf das alleinige Vorliegen eines acetylierten N − Terminus. Die Peptidsequenzen und ihre Spalt − barkeit sind im mittleren Teil der Tabelle in % Spaltung verglichen mit dem Wildtypsub − strat P89 − nach 32 minütiger Inkubation unter den in Beispiel 2 beschriebenen Bedin − gungen − aufgeführt. Die Prozentsätze in Klammern weisen auf die % − Spaltbarkeit des jeweiligen Peptids zu dem Zeitpunkt hin, an dem eine 50 % − ige Spaltung des Wildtyp − substrates P 89 oder E9 erfolgt ist. Der rechte Teil der Tabellen zeigt die Fähigkeit von nicht oder schwach spaltbaren Peptiden als Kompetitoren mit P89 (0,11 mM), bei den angegebenen molaren Verhältnissen, zu wirken.

**Fig. 9:**  Tabelle 3A:

Darstellung der relativen Spaltungseffizienz von substituierten synthetischen Peptiden durch HRV2 2A

a. Kleinbuchstaben bezeichnen ausgetauschte Aminosäuren

b. Kompetitions − Spaltungsreaktionen wurden wie in den Beispielen beschrieben durchgeführt; die Effizienz einer Spaltung wird als $(V_{max}/K_m)_{rel}$ mit P89 als Referenz − peptid ausgedrückt.

Alle Peptide werden an der durch einen Stern markierten Spaltstelle gespalten.

**Fig. 10:**  Tabelle 3B:

Fortsetzung der Tabelle 3A (siehe Figur 10)

**Fig. 11:**  Tabelle 4:

Relative Spaltungseffizienz von synthetischen Peptiden, die von verschiedenen Rhino − und Poliovirus 2A Spaltstellen abgeleitet sind.

a. Ein Punkt zeigt, daß die P' Region aus HRV2 abgeleitet ist;

b. Kleinbuchstaben stellen Aminosäuren dar, die sich von denen aus HRV2 unterschei − den;

c. Kompetitionsspaltungsreaktionen wurden wie in den Beispielen beschrieben durch − geführt; die Spaltungseffizienz wird mit dem Peptid P89 als Referenzpeptid ausge − drückt.

Alle gespaltenen Peptide werden an der durch einen Stern markierten Spaltstelle gespal − ten.

**Abkürzungen**

PMSF:  Phenylmethansulfonylfluorid

TLCK:  Tosyllysylchlormethylketon

| | |
|---|---|
| TPCK: | Tosylphenylalanylchlormethylketon |
| APMSF: | 4 − (Amidinophenyl)methansulfonylfluorid |
| HOBr: | 1 − Hydroxybenzotriazolmonohydrat |
| DIPCDI: | N,N' − Diisopropylcarbodiimid |
| Mtr: | 4 − Methoxy − 2,3,6 − trimethylbenzolsulfonyl |
| Pmc: | 2,2,5,7,8 − Pentamethylchroman − 6 − sulfonyl |
| tBu: | Tert − butyl − |
| Trt: | Trityl − |
| Boc: | Butyloxycarbonyl |
| Fmoc: | 9 − Fluorenylmethoxycarbonyl |
| TFA: | Trifluoressigsäure |
| IPTG: | Isopropylthiogalactosid |
| BSA: | Rinderserumalbumin |
| DMSO: | Dimethylsulfoxid |
| DTT: | Dithiothreitol |

## BEISPIELE

### Allgemeine Methoden

Restriktionsenzyme und DNA modifizierende Enzyme (New England Biolabs oder Boehringer Mann − heim) wurden wie von den Herstellern empfohlen verwendet. Plasmid DNA wurde nach der Methode von Birnboim und Doly (1979, Nucl. Acids Res. 7, 1513 − 1523) präpariert und durch Gelfiltration über Sephacryl S − 1000 gereinigt. DNA Fragmente wurden durch Agarose Gelelektrophorese aufgetrennt und über DE81 − Cellulosepapier isoliert (Maniatis et al. 1982, in: "Molecular Cloning: A Laboratory Manual." Cold Spring Harbor Laboratory, Cold Spring Harbor, New York). Die Sequenzierung erfolgte nach Sanger et al. (1977, Proc. Natl. Acad. Sci. USA 74, 5463 − 5467).

Oligopeptide wurden nach der Fluoren − 9 − ylmethoxycarbonyl (Fmoc) − Strategie mit aktivierten Estern oder HOBt/DIPCDI − Aktivierung auf einem Milligen Modell 9050 Peptid − Synthesizer oder einem Zinsser SMPS 350 Peptid − Synthesizer hergestellt. Als feste Phase zur Anbindung der Peptidsäuren diente Polydimethylacrylamid − Harz auf Kieselgur (Pepsyn KA, Milligen/Biosearch), TentaGel S AC (Rapp Poly − mere), Sasrin − Harz oder Wang − Harz (Bachem, Schweiz) und für Peptidamide TentaGel AM (Rapp Polymere).

Die Fmoc − gekoppelten Aminosäurederivate der freien und der Pentafluorophenylester (Opfp − Ester) stammten von der Bachem AG (Schweiz) und Novabiochem (Schweiz). Die Seitenketten wurden folgen − dermaßen geschützt: Arg (Mtr), Arg (PmC), Asp (tBu), Cys (Trt), Glu (tBu), His (Boc), Ser (tBu) und Thr (tBu). Die Peptide wurden vom Harz durch HF − Behandlung abgespalten und die Seitengruppen durch Inkubation für 3 Stunden in TFA/Thioanisol/Thiocresol (95:3:2, v/v) freigesetzt. Die komplette Entfernung der Mtr − Schutzgruppe gelingt durch eine einstündige Säurebehandlung bei 50˚C. Die Peptide werden in Essigsäure gelöst, in Diethylether präzipitiert, durch Zentrifugation gesammelt und lyophilisiert. Die analy − tische HPLC (Waters System) wurde auf "Bakerbond wide pore C18" − Säulen mit einem linearen Gradienten $H_2O$/TFA (100:0,1) und Acetonitril/TFA (100:0,1) durchgeführt. Semipräparative Trennungen wurden unter den gleichen Bedingungen unter Verwendung einer Hibar Lichsorb RP18 (Merck, Deutschland) gemacht. Die korrekte Aminosäurezusammensetzung aller Peptide wurde mit Hilfe der Plasma − Desorptionsmassen − spektroskopie auf einem BIG ION BIN K20 untersucht.

### Protease Inhibitoren und Chemikalien

Amastatin, Antipain, Bestatin, Chymostatin, E − 64, Elastatinal, Epibestatin, Epiamastatin, Foroxymithin, Iodacetamid, Leupeptin, N − Ethylmaleimid, NLE − Sta − Ala − Sta, Pepstatin, Phosphoramidon, PMSF, TLCK und TPCK wurden von Sigma bezogen. APMSF und 3,4 − Dichloro − isocumarin wurden von Boehringer Mannheim erhalten. 1,10 − Phenantrolin stammte von Merck (Deutschland).

### Beispiel 1: Konstruktion eines IPTG− induzierbaren Expressionsvektors für aktive und inaktive 2A Proteinasen in E. coli HB 101

Das BstEII/HindIII − Fragment von pEx2A (Sommergruber et al., 1989, loc. cit.), das den größten Teil der für die 2A kodierenden Region mit der Ausnahme der ersten 11 N − terminalen Aminosäuren umfaßt, wird isoliert und mit folgendem doppelsträngigen Oligonukleotid ligiert:

$$5' - \quad \text{CATGGGCCCGAGTGACATGTATGTTCATGTAG} \quad -3'$$

$$3' - \quad \text{CCGGGCTCACTGTACATACAAGTACATCCAATG} \quad -5'$$

Das Oligonukleotid umfaßt die fehlende N − terminale kodierende Region der Proteinase 2A einschließlich eines Startkodons. Die Oligonukleotide wurden mit einem "Applied Biosystems DNA Synthesizer" herge − stellt. Die Reinigung erfolgt auf OPC − Säulen (Mc Bride et al., 1988, Biotechniques 6, 362 − 367). Die Oligonukleotide werden mit T4 Polynukleotidkinase phosphoryliert. Für das "Annealing" werden die kom − plementären Oligonukleotide für 10 Minuten auf 68˚C, für 30 Minuten auf 45˚C erwärmt; nach Abkühlung auf Raumtemperatur werden sie auf Eis gestellt.

Das 5' − Ende dieses Oligonukleotids stellt eine "sticky" − NcoI − Schnittstelle, das 3' − Ende eine "sticky" − BstEII − Schnittstelle dar. Das 5' − ATG − Triplett ist als Startkodon Teil der NcoI − Erkennungs − sequenz, die benutzt wird, um die komplette Met − 2A kodierende Region in den mit NcoI und HindIII linearisierten pROK − 1 Expressionsvektor (CLONTECH Laboratories) zu klonieren. Der resultierende Vektor erlaubt die Expression einer reifen Proteinase 2A des HRV2 durch IPTG − Induktion (tac − Promotor) in E. coli HB101 Zellen (ATCC − Nr. 33694). Ein positiver Klon (pROK − 1/2A − 41) wurde mit 2A bezeichnet.

Auf die gleiche Weise werden BstEII/HindIII − Fragmente von pEx2A (Cys106:Ser) und pEx2A (ΔGly107/108) (Sommergruber et al., 1989, loc. cit.) zur Konstruktion von inaktiven 2A Proteinasen benutzt. Die resultierenden Vektoren pROK − 1/2A (Cys106:Ser) und pROK − 1/2A (ΔGly107/108) wurden mit 9B bzw. 1I bezeichnet.

**Beispiel 2: Expression aktiver und nichtaktiver 2A Proteinase und Reinigung mittels MONO O−Säulenchromatographie**

10 ml vorgewärmtes (35˚C) LB − Medium ( + 100 mg Ampicillin /l) werden mit E. coli HB 101 Zellen angeimpft, die entweder den Expressionsvektor für die aktive 2A (pROK − 1/2A − 41) oder für die inaktive Proteinase 2A (pROK − 1/9B und pROK − 1/1I) enthalten, und in einem Inkubationsschüttler bei 35˚C für 8 Stunden ($OD_{600}$: 1,75) inkubiert. Nach Verdünnung durch Zugabe von 10 ml Kulturflüssigkeit zu 90 ml auf Raumtemperatur vorgewärmten LB − Mediums ( + 100 mg Ampicillin/l) wird nach zweistündiger Inkubation ($OD_{600}$: 1,10) die Kultur mit IPTG induziert (0,5 mM Endkonzentration) und für 13 Stunden inkubiert ($OD_{600}$: 2,2). Nach diesem Protokoll liegt ein Maximum an Proteinase 2A löslich vor (ca. 15 bis 20 % der gesamten 2A − Menge). Die Kulturflüssigkeit wird zentrifugiert (10 min, 4000 g, 4˚C) und das Zellpellet aus 50 ml Kulturflüssigkeit in 18 ml eiskaltem 50 mM Tris − HCl (pH 8,5) und 5 mM DTT resuspendiert. Die Zellen werden durch dreimalige Ultraschallbehandlung für je 20 Sekunden (18 ml Aliquots in "Corningtubes" unter Eiskühlung mit einem "MSE ultrasonic power" − Gerät) aufgebrochen. Zur Vermeidung einer Überhitzung wird zwischen den einzelnen Ultraschallbehandlungen eine Pause von je 20 Sekunden gemacht. Zur Abtrennung des unlöslichen Materials wird zunächst in einer Sorvall − Zentrifuge (5 min 5000 g, 4˚C) und anschließend in einer Ultrazentrifuge Beckman, Ti50 Rotor (30 min 35000 g, 4˚C) zentrifugiert. Der Überstand wird auf eine MONO Q 5/5 − Säule gegeben und mittels eines 30 % zu 50 % NaCl − Salzgradienten (Elutionspuffer A: 50 mM Tris − HCl (pH 8,5), 5 mM DTT; Elutionspuffer B: 50 mM Tris − HCl (pH 8,5), 5 mM DTT und 1 M NaCl) eluiert. Fraktionen mit aktiver Proteinase 2A werden durch korrekte Spaltung des Peptidsubstrates "P89" (16 − mer, Beispiel 3), das die Wildtyp Spaltsequenz der HRV2 2A Proteinase repräsentiert, identifiziert (Sommergruber et al. 1989, loc. cit.). Die Hauptaktivität wird bei ca. 45 % des NaCl − Gradierten eluiert. Daneben können Dot Blots zur Identifizierung der 2A enthaltenen Fraktionen gemacht werden. Die Identifizierung inaktiver 2A Proteinase durch Western Blots in MONO Q − Fraktionen erfolgt nach Sommergruber et al. 1989 (loc. cit.) (Fig. 1A und 1B). Die 2A enthaltenden Fraktionen werden vereinigt und auf 8 % Glycerin (Sigma, "molecular biology grade") und 1 mg/ml acetyliertes BSA (Biolabs) eingestellt. In silikonisierten Eppendorfgefäßen werden 0,5 ml Aliquots in flüssigem Stickstoff eingefroren und bei − 20˚C aufbewahrt. Die Aktivität der 2A bleibt über Monate erhalten. Wiederauftauen führt zu einem 5 bis 10 %igen Aktivitätsverlust.

Die Expressionsprodukte von 2A, 9B und 1I wurden nach diesem Protokoll isoliert. Figur 1 zeigt typische MONO Q Elutionsprofile für die Auftrennung der 2A und 9B Extrakte. Die Mutante 1I zeigt ein zu 9B identisches Elutionsprofil. Die korrekte N − terminale Sequenz der 2A enthaltenden Fraktionen wurde durch N − terminale Sequenzierung nachgewiesen.

Met(formyl – Met?) – (Gly) – Pro – Ser – (Asp) – Met – Tyr – Val – $X_{His}$ – Val – $X_{Gly}$ – $X_{Asn}$ –
Leu – Ile – $X_{Tyr}$ –

Die Blockierung eines Großteils des Materials für die N – terminale Sequenzierung legt nahe, daß der überwiegende Teil des 2A Materials ein formyliertes N – terminales Methionin besitzt.

### Beispiel 3: Trans – Spaltungs – Assay: Kinetik der Peptidspaltung mittels teilgereinigter Proteinase 2A

100 $\mu$l der MONO Q – Fraktionen, die aktive Proteinase 2A enthalten, oder 100 $\mu$l der stabilisierten Stammlösungen wurden mit 5 $\mu$l einer wäßrigen Peptidlösung (4 oder 2 mg/ml) gemischt und bei 34˚C für 2, 4, 8, 16 und 32 Minuten inkubiert. Für Kompetitions – oder Inhibierungsstudien werden zusätzlich 5 $\mu$l Lösung, die die Substanz zur Kompetition oder Inhibierung enthält, zugegeben. Die Reaktion wird durch Zugabe eines gleichen Volumens einer 0,5 M $HClO_4$ – Lösung gestoppt. Die Proben werden für 10 Minuten auf 0˚C gekühlt oder über Nacht bei – 20˚C eingefroren. Nach Zentrifugation (Eppendorf – Zentrifuge, 10 min, 4˚C) wird ein gleiches Volumen einer 1,4 M $K_2HPO_4$ – Lösung zugegeben. Die Proben werden für 5 Minuten bei Raumtemperatur stehengelassen und das Präzipitat durch Zentrifugation abgetrennt. Der Überstand wird auf eine "Reverse – phase" HPLC – Säule gegeben und die Peptidprodukte entweder mittels eines kurzen Gradienten (Merck Supersphere C18 Säule) oder eines langen Gradienten (Bakerbond WP C18 Säule) mit wäßriger 0,1 % Trifluoressigsäure und Acetonitril als mobiler Phase aufgetrennt. Die Detektion der Reaktionsprodukte erfolgt durch UV – Absorption bei 214 und 280 nm. Der zeitliche Reak – tionsverlauf (% Spaltung des Peptidsubstrates) wird mit "Reverse phase" HPLC bestimmt. Die Fläche der neugebildeten Peptidpeaks, die der Menge der Carboxy – und aminoterminalen Spaltprodukte entsprechen, wird für jeden Fall bestimmt und mit der Fläche des ungespaltenen P89 verglichen. Die Spaltungsprodukte werden entweder durch Komigration mit den entsprechenden Referenzpeptiden und Aminosäureanalyse oder durch Proteinsequenzierung (Hunkapillar and Hood, 1983, loc. cit.) bestimmt.

Wie in Beispiel 2 beschrieben, wird die aktive Proteinase 2A mittels eines 16 Aminosäure langen Wildtyp Oligopeptidsubstrates "P89" (acetyl – TRPIITTA*GPSDMYVH – $NH_2$) identifiziert, das spezifisch nach dem Aminosäurepaar Ala – Gly gespalten wird (Fig. 1A), während weder die Cys106:Ser Mutante 9B noch die ΔGly107/108 Mutante 1I eine spezifische Spaltung des Peptidsubstrates P89 bewirken (Fig. 1B). Aufgrund der Präsenz endogener Proteinasen in E. coli wird gewöhnlich eine geringe unspezifische Spaltung von P89 zwischen Ile – Ile und/oder Arg – Pro in ungereinigten Extrakten beobachtet. Nach Reinigung mit MONO Q Säulen wird diese unspezifische Spaltungsaktivität eindeutig von 2A – enthaltenden Fraktionen abgetrennt (eine unspezifische Spaltung wird nur in den MONO Q Fraktionen 1 bis 3 nachge – wiesen; Fig. 1). Außerdem wird keine Hintergrundaktivität (spezifisch oder unspezifisch) in Fraktionen festgestellt, die das Expressionsprodukt von 9B (Fig. 1B) oder 1I enthalten. In allen anderen Studien dienen diese teilgereinigten 9B – und 1I – Fraktionen als Negativkontrolle. Der Gebrauch einer Vielzahl verschie – dener Peptidsubstrate zeigt, daß keine Spaltung (spezifisch oder unspezifisch) in teilgereinigten 9B – oder 1I – Extrakten auftritt. Durch diese Daten kann eine gleichzeitige Mitreinigung von interferierenden E. coli – Proteinasen ausgeschlossen werden.

### Beispiel 4: Einfluß des pH – Wertes auf die proteolytische Trans – Aktivität

100 $\mu$l frisch präparierte, nicht stabilisierte 2A enthaltende MONO Q Fraktionen (25 mM Tris – HCl (pH 8,5), 5 mM DTT, 450 mM NaCl) als auch gefrorene, stabilisierte Aliquots (25 mM Tris – HCl (pH 8,5), 5 mM DTT, 450 mM NaCl, 8 % Glycerin und 1 mg/ml acetyliertes BSA) wurden durch Zugabe von 10 $\mu$l einer 0,5 M Lösung verschiedener Pufferlösungen auf unterschiedliche pH – Werte eingestellt (Fig. 2). Um jeden inhibitorischen Effekt der ausgewählten Puffer zu vermeiden und um Effekte auszuschließen, die aus der unterschiedlichen Ionenstärke eines Puffersystems resultieren, werden verschiedene Puffersysteme benutzt: Glycin/HCl, Glycin/NaOH, Natriumcitrat/HCl, Phthalsäure/NaOH, Tris/HCl, $NaH_2PO_4$/$Na_2HPO_4$ und $CH_3COOH$/NaOH. Nach 10 minütiger Inkubation bei Raumtemperatur wird der pH – Wert mit einer Mikro – elektrode (Ingold) gemessen und kinetische Studien wie in Beispiel 3 beschrieben, durchgeführt. Für die Proteinase 2A ergibt sich ein pH – Optimum zwischen pH 7 und pH 8.5.

Um die Stabilität dieses Enzyms zu untersuchen, wird die Proteinase 2A bei unterschiedlichen pH – Werten für 20 Minuten bei Raumtemperatur vorinkubiert, gefolgt von einem Nachstellen des pH – Wertes zwischen pH 8 und pH 8,5 durch Zugabe von 1/20 Volumen einer 2M Tris – HCl – Lösung (pH 8,5). Anschließend wurde der Trans – Spaltungs – Assay durchgeführt. Im Vergleich zum ersten Test zeigt 2A eine größere Toleranz bei sauren pH – Werten, während kleinere Änderungen im basischen Bereich zu einer größeren Sensitivität führen. (Fig. 2). Die Zeichnung in Figur 2 zeigt, daß die Proteinase 2A bei kleineren pH−Werten nicht irreversibel zerstört wird; bei Re−Titrationen von pH−Werten zwischen 4 und 9 auf pH 8,5 erhält sie die volle Aktivität zurück. Da diese Kurve die Stabilität der Proteinase 2A darstellt, treten offensichtlich kinetisch wichtige Ionisierungen eher an sauren als an basischen Seitengruppen auf.

**Beispiel 5: Bestimmung des Km−Wertes bei Verwendung des Peptidsubstrates P89**

Zur Identifizierung des Temperaturoptimums wird ein Satz von Trans−Spaltungs−Assays durchgeführt. Proben, die die Proteinase 2A enthalten, werden vor kinetischen Untersuchungen für 10 Minuten bei der gewünschten Temperatur inkubiert. Weiterhin wird der Temperaturbereich identifiziert, in dem 20 % der Spaltung in den ersten 5 bis 10 Minuten einen linearen Verlauf zeigen. Die so identifizierte Anfangssteigung wird zur Km−Bestimmung mit P89 als Substrat benutzt. Die Temperaturkurve zeigt einen linearen Bereich der proteolytischen Aktivität zwischen 5°C und 20°C, gefolgt von einem Plateau bei 20°C bis 35°C und einem steilen Abfall bei 37°C. Der geeignete Meßbereich mit linearem Verlauf wird bei 15°C festgestellt.

Zur Identifizierung des Km−Wertes für das Wildtypsubstrat P89 wird ein Satz von Trans−Spaltungs−Assays bei 15°C mit verschiedenen P89 Substratkonzentrationen (zwischen 50 und 500 $\mu$mol/l) durchge−führt. Proben werden in halbminütigen Intervallen bis zu einer Gesamtdauer von 3 Minuten gezogen. Für jede Substratkonzentration wurde die Reaktionsgeschwindigkeit (% Spaltung von P89) bestimmt. Das Experiment wird dreimal mit verschiedenen 2A Präparationen wiederholt. Es ergibt sich ein Km−Wert von $(5,4 \pm 0,02) \times 10^{-4}$ mol/l.

**Beispiel 6: Effekt von bekannten Proteinaseinhibitoren auf die Spaltung des Peptids P89**

Der Effekt einer Reihe bekannter Inhibitoren von Serin−, Cystein−, Aspartat− und Metalloproteinasen, als auch der Effekt reduzierender Agentien auf die Aktivität der 2A gegenüber dem Peptid P89 wird untersucht. Die Trans−Spaltungs−Assays werden durchgeführt wie beschrieben, nur daß die verschiedenen Proteinaseinhibitoren vor Reaktionsbeginn zugegeben werden. Die kinetischen Untersuchungen, die mit frisch präparierten und stabilisierten 2A−Präparationen dreimal wiederholt werden, zeigen einen hohen Grad an Reproduzierbarkeit. Die Resultate sind in Tabelle 1 zusammengefaßt. Werden DMSO, Methanol oder Ethanol zur Lösung der Inhibitoren benötigt, werden sie entsprechend der benötigten Menge auch den Kontrollen zugegeben. Weiterhin werden zur Untersuchung SH−sensitiver Inhibitoren dialysierte 2A−Präparationen eingesetzt (gegen 50 mM Tris−HCl, pH 8,5, und 400 mM NaCl). Die hier verwendeten Bedingungen und effektiven Konzentrationen der Inhibitoren werden wie beschrieben eingesetzt (Beynon und Salvesen, 1989, loc. cit).

**Beispiel 7: Rolle der Peptidlänge für 2A**

Eine Reihe von Peptiden wird zur Bestimmung der Mindestpeptidlänge für die Spaltung durch die HRV2 Proteinase 2A synthetisiert. Es wird gezeigt, daß ein 16 Aminosäuren langes Peptid (P89), das die Region zwischen dem C−Terminus von VP1 und dem Aminoterminus von 2A als effektives Substrat für die Proteinase 2A dienen kann (Sommergruber et al., 1989, loc. cit.). Die Substratfähigkeit kürzerer Peptide wird mittels Trans−Spaltungs−Assays überprüft (unter identischen Bedingungen wie in Beispiel 3 beschrieben) und mit P89 verglichen. Alle Peptide dieser Untersuchung enthalten acetylierte N− und amidierte C−Termini. Die Ergebnisse sind in Figur 4 zusammengefaßt. Sukzessive C−terminale Deletionen innerhalb P89 (Peptide E9, WT14, WTC13, WTD12, CWT10, und WTN9) haben weit weniger Einfluß auf die Spaltbarkeit, als N−terminale Deletionen (Peptide A, B, C, D und E). Längenreduktion des amino−terminalen Endes von P89 über die Aminosäure Prolin (Position P6) führt zu einem Abfall der Spaltbarkeit um eine Größenordnung. Offensichtlich ist dieser Teil des Peptides kritisch für eine effektive Erkennung und Spaltung (Peptide C und D). Demgegenüber ist jedoch die Mindestzahl von einer Aminosäure (Peptid WTN9) auf der carboxy−terminalen Seite für eine 10 %ige Spaltung ausreichend. Demzufolge spielt das C−terminale Ende eine kleinere Rolle für die Substraterkennung und/oder Spaltung. Abbau von beiden Seiten des Peptidsubstrates führt zu einem 11−mer Substrat (WTC11), das noch effizient gespalten werden kann (40 %). Weiterer Abbau von beiden Seiten erlaubt keine Spaltung mehr. Eine Löslichkeitsänderung als Ursache für Unterschiede in der Spaltbarkeit wurde unter den Versuchsbedingungen nie beobachtet.

**Beispiel 8: Intermolekulare Substratsspezifität synthetischer Peptidsubstrate und Inhibierung der P89‑Spaltung durch modifizierte Peptidsubstrate**

Rhinovirale 2A Proteinasen erkennen ein Ala oder Val an Position P1 und ein Gly an Position P1', mit Ausnahme der Proteinase 2A von HRV14, die eine Tyr‑Gly Bindung spaltet, ähnlich wie bei den Serotypen des Poliovirus. Coxsackie Viren besitzen eine Thr‑Gly Spaltungssequenz an ihrer VP1‑2A Schnittstelle. Die meisten Spaltungssequenzen der Rhino‑ und Enteroviren besitzen ein Thr an Position P2 und/oder P3. Nur HRV14 und HRV9 (Kräusslich und Wimmer, 1988, loc. cit.) weisen kein Thr an einer dieser Positionen auf (Stanway et al. 1983, loc. cit.; Lecki, G.W. 1988, loc. cit.). Weiterhin besitzen alle bekannten Rhinovirus‑Stämme ein Prolin an Position P2' mit der Ausnahme von HRV14. Um mehr über die unterschiedliche Verteilung der Aminosäuren für die Substratspezifität der Transaktivität der 2A zu verste‑ hen, wird ein Satz von 15‑mer Peptiden synthetisiert, die einzelne Mutationen an den Positionen P2, P1, P1', P2' und P3' tragen (Fig. 4). Die ursprüngliche Länge von P89 (16 Aminosäuren) wird verkleinert (15 Aminosäuren), weil dies eine bessere Trennung der verschiedenen Peptidsubstrate und ihrer Spaltungsro‑ dukte auf dem kleinen HPLC‑Gradienten erlaubt und nach den vorliegenden Daten über die Verkürzung der Peptidsubstrate keinen Einfluß auf die Kinetik der Proteolyse hat.

Mit diesen modifizierten Peptidsubstraten werden Trans‑Spaltungs‑Assays wie beschrieben durch‑ geführt. Um einen Vergleich der Spaltungseffizienz der mutierten Peptide zu ermöglichen, wird ein 15‑mer Wildtyp‑Substrat (E9, Tabelle 2) synthetisiert, das genauso effizient gespalten wird wie das 16‑mer Wildtypsubtrat P89 (Tabelle 2 und Fig. 4). Um jeden unphysiologischen Einfluß der Ladung, die durch freie N‑ oder C‑terminale Aminosäuren auftritt, auszuschließen, werden einige der Wildtypsubstrate ein‑ schließlich P89 an ihrem N‑Termini acetyliert und an ihren C‑Termini carboxyliert (in Fig. 4 durch ein offenes Dreieck gekennzeichnet). Dies gilt besonders für die Ladungsverteilung kleinerer Peptide unter 10 Aminosäuren. Es stellt sich jedoch für die 15 und 16 Aminosäure‑langen Peptide heraus, daß sie, unabhängig davon ob sie acetyliert und amidiert sind, keinen signifikanten Unterschied in ihrer Spaltbarkeit aufweisen. Für weitere Untersuchungen wird diese Modifikation weggelassen. In Fällen, in denen Spaltung beobachtet wird, werden die auftretenden Spaltstellen durch N‑terminale Aminosäuresequenzierung oder durch Komigration mit entsprechenden Referenzpeptiden festgestellt. Die Mutationsanalysen zeigen hohe Toleranz gegen Austausche an den Positionen P1, P2' und P3', jedoch eine absolute Abhängigkeit vom Threonin an P2 und von Glycin an P1' (Tabellen 2A und 2B). HRV2 2A toleriert an der Position P2 nur Austausche von Aminosäuren, die an Größe bzw. Polarität dem Thr am nächsten kommen (Peptide B4, B8 und B12; Tabelle 2A). Positiv geladene Reste in Position P2 (Peptide C3 und C5) zeigen eine (wenn auch stark reduzierte) Spaltbarkeit. Eine mögliche Interpretation der Akzeptanz von positiven Ladungen an dieser Position wäre die strukturelle Ähnlichkeit zu Trypsin‑ähnlichen Proteinasen (Bazan und Fletterick, 1990, loc. cit.), die bekanntermaßen nach basischen Aminosäuren spalten. Alle anderen Mutationen im Peptid‑ substrat führen dazu, daß das Peptid nicht mehr von 2A gespalten werden kann (Tabelle 2A). Die Position P2 scheint daher (neben dem ITTA‑Motiv und dem Arg in Position P7) eine essentielle Rolle zu spielen.

Durch Vergleich der %‑Spaltbarkeit der Peptidmutanten zu einem Zeitpunkt, bei welchem 50% Spaltung des Wildtypsubstrates (P89 und E9) vorliegt, konnte gezeigt werden, daß bei Anwesenheit eines Met oder Tyr in Position P1 (Peptide F10, H2, P146 und F) eine höhere Effizienz an Spaltbarkeit gegeben ist (Tabelle 2A). Diese beiden Reste in P1 dürfen für die HRV2 2A eine kinetisch günstigere Situation für die Trans‑Aktivität schaffen.

Weiterhin werden Deletionen in das Wildtypsubstrat eingeführt, (Peptide AB1, AB2 und ST2), um zu überprüfen, ob die HRV2 2A auch andere, weniger dominante Spaltsignale innerhalb eines Peptids, wie sie für die cis‑Aktivität von Polio 2A (Hellen et al., 1989, loc. cit.) beschrieben wurden, akzeptiert. Keine Spaltung (auch keine alternative) wird für AB1, AB2 und ST2 beobachtet (Tabelle 2C). Die Einführung von D‑Aminosäureanaloga bei P2, P1 und P2' (Peptide P2dT, C11, H10 und P2'dP) erlaubte keine Spaltung der modifizierten Substrate, was eindeutig für die stereochemische Spezifität der HRV2 2A spricht (Tabelle 2B). Eine CARBA‑Substitution des Spaltsignals durch ε‑Aminocapronsäure oder Statin (Peptide Capro und STAT) führten zu einem kompletten Verlust der Spaltbarkeit (Tabelle 2B).

Ausgehend von der Spaltstelle VP1‑2A und der alternativen Stelle innerhalb 3D des Polioserotyps 1 (Lee und Wimmer, 1988, loc. cit.) wurden Peptidsubstrate (P73 und 75) und ihre C‑terminalen Spaltpro‑ dukte (P74 und P76) synthetisiert.

Obgleich hier ein extremer Unterschied zwischen der Primärsequenz der Spaltstelle von Polio und Rhino vorliegt, wird die VP1‑2A Spaltsequenz von Polio durch die HRV2 2A Proteinase in geringem Ausmaß akzeptiert, was auf eine, wenn auch minimale, strukturelle Gemeinsamkeit zwischen P89 und P73 hinweist. Die alternative Polio‑Region in 3D (Peptid 75) dient nicht als Substrat für die HRV2 2A (Tabelle 2C).

Chimäre Peptidsubstrate bestehend aus einem N‑Terminus von HRV1A, HRV1B, HRV39 oder HRV49 (Palmenberg, A., in Molecular Aspects of Picornavirus Infection and Detection, pp. 211‑231; ed. by. B. L. Semler and E. Ehrenfeld, Washington, D.C.: American Society for Microbiology; Hughes et al., loc. cit.) und aus einem C‑terminalen Bereich, der von HRV2 (Skern, et al., loc. cit.) abgeleitet ist (Tabelle 2C; Peptide RV1A, RV1B, RV39 und RV49), zeigen die besondere Bedeutung des ITTA‑Motivs und/oder des Arg in Position P7. Die Peptide RV9, RV14, RV85 und RV89 (Lecki, G.W. 1988, loc. cit.; Stanway et al., 1984, loc. cit.; Callahan, 1985, loc. cit.; Stanway, G. 1990, loc. cit. und Duechler et al., loc. cit.) repräsentieren die Wildtyp‑Spaltstelle der entsprechenden Serotypen. Die Peptide RV9, RV14 und RV89, die den größten Unterschied in der Aminosäuresequenz aufweisen (weder ein ITTA‑Motiv noch ein Arg an Position P7 ist vorhanden) können überhaupt nicht gespalten werden; die Peptide RV39, 1A und 1B können im Vergleich zu P89 zu ca. 10 % und die Peptide RV85 und 49 können mit guter Effizienz (ca. 50 %) gespalten werden. Dieses Resultat zeigt wieder, daß der N‑terminale Bereich der Peptide (besonders das ITTA‑Motiv und/oder das Arg in Position P7) entscheidend für eine Spaltung ist. Dies wurde auch durch die verkürzten Wildtyp‑Peptide gezeigt (Fig. 4).

Einige P2‑substituierte Peptide konnten mit 2A kopräzipitiert werden, was zeigt, daß diese Peptide fest an der Proteinase 2A, möglicherweise in der Nähe des aktiven Zentrums, gebunden, aber nicht gespalten werden. Damit stellt sich die Frage, ob diese oder andere nicht oder nur schwach spaltbare Peptide kompetitive Inhibitoren von P89 sind. Aus der Fähigkeit von nicht oder schwach spaltbaren Peptiden als kompetitive Inhibitoren zu agieren, läßt sich weiter ableiten, ob eine Aminosäure des Peptidsubstrates an der Substratbindung beteiligt ist, oder direkt mit dem katalytischen Zentrum der 2A wechselwirkt. Zur Erhöhung der Konzentration der verschiedenen Peptidsubstrate wurden Aliquots der Stammlösung (4 mg bzw. 2 mg/ml) lyophilisiert und die Pellets direkt in dem entsprechenden Volumen der die 2A enthaltenden Proben gelöst. Nur diejenigen getesteten Peptide verhielten sich kompetitiv, die entweder von verkürzten oder deletierten Wildtyp‑Peptidsubstraten (P145, WTD8 und AB2; Tabelle 2C) oder von Peptiden mit "moderaten" Aminosäureaustauschen an Position P1 oder P2 (Ala gegen Asn, Peptid G5, oder Thr gegen Ser oder Asn, Peptide B4 und B8) abgeleitet waren. Ein mutiertes Peptid mit einem Aminosäureaustausch Gly gegen Thr an Position P1' konnte nicht gespalten werden, es zeigte jedoch eine eindeutige, wenn auch geringe, Inhibition, die besagt, daß diese Aminosäureposition eine untergeordnete Rolle in der Substrater‑ kennung spielt, aber offensichtlich sterisch bedeutsam für die Spaltung ist. Das gleiche gilt für Peptide die eine CABRA‑Substitution anstelle des HRV2‑Spaltsignals Ala‑Gly enthalten (Tabelle 2C; Peptide Capro und STAT).

**Beispiel 9: Spaltungsassays für die $(V_{max}/K_m)_{rel}$‑Wertbestimmung**

Die Spaltungsassays wurden wie oben beschrieben mit einer Mischung aus dem Standardpeptid P89 und dem jeweiligen Testpeptid (jeweils 100 $\mu$M) durchgeführt. Aliquots wurden in halbminütigen Intervallen bis zu vier Minuten und anschließend nach 8, 16 und 32 Minuten entnommen und wie oben beschrieben behandelt. Die Peptide wurden wie oben beschrieben mittels HPLC analysiert. Die Analyse der erhaltenen Peakflächen erlaubte die Bestimmung des Ausmaßes der Substratumwandlung. Die erhaltenen Daten wurden wie von Pallai et al. (J. Biol. Chem. (1989) 264, 9738‑9741) beschrieben zur Bestimmung der $(V_{max}/K_m)_{rel}$‑Werte ausgewertet. Der Wert abgeleitet für die Substrate 1 und 2 beträgt unter Verwendung von

$$(V_{max}/K_m)_1/(V_{max}/K_m)_2 = \log(1-F_1)/\log(1-F_2)$$

wobei F die umgewandelte Substratfraktion darstelt. In allen durchgeführten Kompetitionsassays waren Substrat‑ und Produktpeptide voneinander trennbar. Alle Peptide von denen keine Produkte innerhalb von 32 Minuten erhalten wurden, wurden mit HRV2 2A für insgesamt 3 Stunden inkubiert. Traten nach dieser Zeit keine Produkte auf, wurden die Substrate als "nicht gespalten" definiert.

Die $(V_{max}/K_m)_{rel}$‑Werte sind in Fig. 9 bis 11 dargestellt.

**Patentansprüche**

1. DNA‑Vektor, enthaltend eine für eine HRV Proteinase 2A kodierende Sequenz sowie die durch Mutation, Deletion und/oder Insertion herstellbaren Varianten, die für eine enzymatisch aktive oder nicht aktive HRV 2A Proteinase kodieren, dadurch gekennzeichnet, daß er die Expression des reifen Proteins erlaubt.

2. DNA – Vektor gemäß Anspruch 1, dadurch gekennzeichnet, daß er die Expression einer HRV2 2A Proteinase erlaubt.

3. DNA – Vektor gemäß Anspruch 1 – 2, dadurch gekennzeichnet, daß er pROK – 1/2A – 41, pROK – 1/I1 oder pROK – 1/9B ist.

4. Wirtsorganismus, dadurch gekennzeichnet, daß er einen Vektor gemäß den Ansprüchen 1 – 3 enthält.

5. Wirtsorganismus gemäß Anspruch 4, dadurch gekennzeichnet, daß er E. coli, vorzugsweise E. coli HB 101 ist.

6. Verfahren zur Expression einer HRV 2A Proteinase, dadurch gekennzeichnet, daß
   a. ein Wirtsorganismus gemäß den Ansprüchen 4 – 5 mit einem Vektor gemäß den Ansprüchen 1 – 3 transformiert,
   b. die Proteinase 2A zur Expression gebracht wird und gereinigt wird.

7. Verfahren gemäß Anspruch 6, dadurch gekennzeichnet, daß die Reinigung mittels anionischer Ionen – austauschchromatographie, bevorzugt an MONO Q – Material, durchgeführt wird.

8. Peptid und abgeleitete chemische Derivate, mit inhibitorischer Wirkung gegen die rhinovirale HRV2 2A Protease, abgeleitet von dem Aminosäuremotiv TRPIITTAGPSDMYVH, dadurch gekennzeichnet, daß es durch N – oder C – terminale Verkürzung erhalten wird und/oder das ITTAG – Motiv verändert ist.

9. Peptid gemäß Anspruch 8, dadurch gekennzeichnet, daß
   a. ein oder mehrere Aminosäuren deletiert sind und/oder
   b. daß es einen moderaten Aminosäureaustausch an Position P1 und/oder Position P2 enthält und/oder
   c. daß es an Position P2 eine Aminosäure mit einer reaktiven Seitenkette und/oder
   d. anstelle der Ala – Gly – Spaltstelle C4 – oder C6 – Aminosäuren und/oder
   e. anstelle des P1 – und/oder P2 – Restes $\gamma$ – Amino – $\beta$ – hydroxybuttersäure enthält.

10. Peptid gemäß Anspruch 9, dadurch gekennzeichnet, daß es N – oder C – terminal verkürzt ist und/oder eine Deletion der Positionen P1 und/oder P1' aufweist.

11. Peptid gemäß Anspruch 10, dadurch gekennzeichnet, daß es P145(IITTAGPSDM), WTD8(ITTAGPSD) oder AB2(TRPIITTPSDMYVH) ist.

12. Peptid gemäß Anspruch 9, dadurch gekennzeichnet, daß ein moderater Aminosäureaustausch ein Asn an Position P1 und/oder ein Ser oder Asn an Position P2 umfaßt.

13. Peptid gemäß Anspruch 12, dadurch gekennzeichnet, daß es G5(TRPIITTNGPSDMYV), B4(TRPIITSAGPSDMYV) oder B8(TRPIITNAGPSDMYV) ist.

14. Peptid gemäß Anspruch 9, dadurch gekennzeichnet, daß die Aminosäure mit einer reaktiven Seiten – kette an Position P2 $\alpha$ – Aminobuttersäure, Vinylglycin, Homocystein, Homoserin, $\beta$ – Cyanoalanin oder S – Methylcysteinsulfoxid ist.

15. Peptid gemäß Anspruch 9, dadurch gekennzeichnet, daß die Ala – Gly – Spaltstelle bzw. die Amino – säurepositionen P1 und P2 durch $\delta$ – Aminolävulinsäure, $\gamma$ – Aminobuttersäure oder Ornithin, $\alpha$ – Amino – $\beta$ – hydroxybuttersäure, $\delta$ – Aminocapronsäure oder Statin ersetzt ist.

16. Peptid gemäß einem der Ansprüche 8 – 15, dadurch gekennzeichnet, daß zur Erhöhung der Peptidaf – finität die Aminosäure an Position P1 Methionin oder Tyrosin ist.

17. Arzneimittel gemäß Anspruch 8 – 16, gegebenenfalls mit Hilfs – und/oder Trägerstoffen und/oder Stabilisatoren, zur Inhibierung der HRV 2A Proteinase.

19

**18.** Verwendung eines Peptids gemäß den Ansprüchen 8 – 16 zur Herstellung eines Arzneimittels zur Inhibierung der HRV 2A Proteinase.

**19.** Mittel enthaltend ein Peptid gemäß den Ansprüchen 8 – 16, gegebenenfalls mit Hilfs – und/oder Trägerstoffen und/oder Stabilisatoren, zur Inhibierung der HRV 2A Proteinase.

**20.** Verfahren zur Herstellung der Peptide gemäß den Ansprüchen 8 – 16, dadurch gekennzeichnet, daß sie nach der Fmoc – Strategie mit aktivierten Estern oder über HOBt/DIPCDI – Aktivierung sowie nach Analogieverfahren hergestellt werden.

Fig. 1A

Fraktions-Nr.

Fig. 1B

B

A$_{280}$

% Aktivität

1.25

1.0 — 0.5 M NaCl — 100

0.75 — 80

0.3 M NaCl

0.5 — 60

0.25 — 40

— 20

1   2   3   4   5   6   7   8   9   10  11  12  13  14

Fraktions-Nr.

C    1  2  3  4  5  6  7  8  9  10 11 12 13 14

←58
←48

←36

←26

9B →                                    ← 15

Fig. 2

Fig. 3

EP 0 541 058 A1

Fig. 4

Fig. 5

Tabelle 1

| Inhibitor Class | Inhibitor | $IC_{50}$ (µmol/l) |
|---|---|---|
| Metallo Protease | Epiamastatin<br>Foroxymithin<br>Phosphoramidon<br>Amastatin<br>Bestatin<br>Epibestatin<br>EGTA<br>EDTA<br>1,10 Phenanthroline | -<br>-<br>-<br>-<br>-<br>-<br>-<br>16 500<br>3 200 |
| Asp Protease | NLE-Sta-Ala-Ala<br>Pepstatin | -<br>- |
| Ser Protease | 3,4-dichloro-isocoumarin<br>Elastatinal<br>APMSF<br>TLCK<br>TPCK<br>PMSF | 1430<br>55<br>-<br>80<br>1300<br>1200 (±500)* |
| Cys Protease | E-64<br>Iodacetamide<br>NEM | -<br>90<br>28 |
| Cys/Ser Protease | Antipain<br>Leupeptin<br>Chymostatin<br>pCMPSA | 65<br>-<br>23<br>>50 ** |
| Reducing Agents | DTT<br>Cysteine | -<br>- |

26

EP 0 541 058 A1

Tabelle 2A

Fig. 6

| # | Sequenz | Gespalten (%) | Molares Verhältnis P89: Peptid/Kompetition (%) | | | |
|---|---------|---------------|------|------|------|--------|
| | | | 1:4 | 1:2 | 1:1 | 1:0.5 |
| P1- und P2-substituierte Peptidsubstrate | | | | | | |
| P89 | Δ  T R P I I T T A * G P S D M Y V H | 100(50) | <5 | 0 | 0 | 0 |
| E9 | T R P I I T T A * G P S D M Y V | 100(50) | 0 | 0 | 0 | 0 |
| F2 | T R P I I T T (L) * G P S D M Y V | 49(27) | | | | |
| F6 | T R P I I T T V * G P S D M Y V | 28(7) | | | | |
| F8 | T R P I I T T P  G P S D M Y V | 0 | — | <5 | 0 | 0 |
| F10 | T R P I I T T M * G P S D M Y V | 100(91) | | | | |
| F12 | T R P I I T T T * G P S D M Y V | 98(49) | | | | |
| G5 | T R P I I T T N * G P S D M Y V | 16(10) | 75 | — | <5 | 0 |
| G7 | T R P I I T T Q * G P S D M Y V | 39(20) | | | | |
| G9 | T R P I I T T E  G P S D M Y V | 0 | — | — | 0 | 0 |
| G11 | T R P I I T T D  G P S D M Y V | 0 | — | 0 | 0 | 0 |
| H2 | T R P I I T T Y * G P S D M Y V | 100(51) | — | 0 | 0 | — |
| H4 | T R P I I T T K * G P S D M Y V | 19(8) | | | | |
| H6 | T R P I I T T R * G P S D M Y V | 55(24) | | | | |
| H12 | T R P I I T T F * G P S D M Y V | 92(40) | | | | |
| P146 | Δ  T R P I I T T Y * G P S D M Y V H | 100(66) | | | | |
| F | Δ I V T R P I I T T (Y) * G P S D M Y V H | 100(63) | | | | |
| A1 | T R P I I T (G) A  G P S D M Y V | 0 | — | <5 | 0 | 0 |
| A3 | T R P I I T V A  G P S D M Y V | 0 | — | <5 | 0 | 0 |
| A5 | T R P I I T A A  G P S D M Y V | 0 | — | <5 | 0 | 0 |
| A7 | T R P I I T L A  G P S D M Y V | 0 | — | <10 | 0 | 0 |
| A9 | T R P I I T I A  G P S D M Y V | 0 | — | — | 0 | 0 |
| A11 | T R P I I T P A * G P S D M Y V | 5(>1) | — | <10 | <5 | 0 |
| B4 | T R P I I T S A * G P S D M Y V | 9(4) | 55 | 30 | 15 | 0 |
| B6 | T R P I I T H A * G P S D M Y V | <1(0) | — | 0 | 0 | 0 |
| B8 | T R P I I T N A * G P S D M Y V | 30(15) | — | <15 | 5 | 0 |
| B10 | T R P I I T Q A * G P S D M Y V | 12(4) | — | — | 5 | 0 |
| B12 | T R P I I T E A  G P S D M Y V | 0 | — | 0 | 0 | 0 |
| C1 | T R P I I T D A  G P S D M Y V | 0 | — | 0 | 0 | 0 |
| C3 | T R P I I T K A  G P S D M Y V | 5(3) | — | >5 | 5 | 0 |
| C5 | T R P I I T R A * G P S D M Y V | 7(>1) | — | — | 0 | 0 |
| C7 | T R P I I T Y A * G P S D M Y V | <1(0) | <10 | <5 | 0 | 0 |
| D2 | T R P I I T (F) A  G P S D M Y V | 0 | — | 0 | 0 | 0 |

EP 0 541 058 A1

| # | Sequenz | Gespalten (%) | Molares Verhältnis P89: Peptid/Kompetition (%) | | | |
|---|---------|---------------|:---:|:---:|:---:|:---:|
| | | | 1:4 | 1:2 | 1:1 | 1:0.5 |
| **Pl'-, P2'- und P3'- substituierte Peptidsubstrate** | | | | | | |
| P89 | Δ   T R P I I T T A * G P S D M Y V H | 100(50) | <5 | 0 | 0 | 0 |
| E9 | T R P I I T T A * G P S D M Y V | 100(50) | 0 | 0 | 0 | 0 |
| D4 | T R P I I T T A (F) P S D M Y V | 0 | – | 0 | 0 | 0 |
| D6 | T R P I I T T A D P S D M Y V | 0 | – | – | <5 | 0 |
| D8 | T R P I I T T A * K P S D M Y V | 0 | – | – | <5 | 0 |
| D10 | T R P I I T T A (T) P S D M Y V | 0 | – | 15 | <10 | 0 |
| D12 | T R P I I T T A * G (F) S D M Y V | 32(10) | | | | |
| E1 | T R P I I T T A * G D S D M Y V | 26(12) | | | | |
| E3 | T R P I I T T A * G K S D M Y V | 35(21) | – | 40 | 20 | – |
| E5 | T R P I I T T A * G (T) S D M Y V | 37(19) | | | | |
| C9 | T R P I I T T A * G P (D) D M Y V | 38(8) | | | | |
| E7 | T R P I I T T A * G P T D M Y V | 94(41) | | | | |
| H8 | T R P I I T T A * G P (K) D M Y V | 92(43) | | | | |
| **D-Aminosäure- und CARBA- substituierte Peptidsubstrate** | | | | | | |
| P2dT | T R P I I T (t) A   G P S D M Y V H | 0 | – | 0 | 0 | 0 |
| C11 | T R P I I T (a) A   G P S D M Y V H | 0 | 15 | <5 | 0 | 0 |
| H10 | T R P I I T T (a)   G P S D M Y V H | 0 | – | 0 | 0 | 0 |
| P2'dP | T R P I I T T A   G (p) S D M Y V H | 0 | <5 | 0 | 0 | 0 |
| Capro | T R P I I T T (ε-cap) P S D M Y V H | 0 | 15 | <5 | 0 | 0 |
| STAT | T R P I I T T (stat) P S D M Y V H | 0 | – | <15 | <10 | – |
| **Referenzpeptide (Spaltungsprodukte)** | | | | | | |
| 89/12 | ● T R P I I T T A | 0 | 10 | <5 | 0 | 0 |
| 89/13 | ○              G P S D M Y V H | 0 | – | 0 | 0 | 0 |
| UP | T R P I I T T A | 0 | >5 | <5 | 0 | 0 |
| DOWN |              G P S D M Y V | 0 | – | 0 | 0 | – |
| P46 | ●      I T T A | 0 | – | 0 | 0 | 0 |
| P47 | ○              G P S | 0 | – | 0 | 0 | 0 |

Fig. 7

# Tabelle 2C  Fig. 8

**2A-Substrate von Polio (PV1) und verschiedenen Rhinoserotypen**

| # | | Sequenz | Gespalten (%) | Molares Verhältnis P89: Peptid/Kompetition (%) 1:4 | 1:2 | 1:1 | 1:0.5 |
|---|---|---|---|---|---|---|---|
| P89 | △ | TRPIITTA * GPSDMYVH | 100(50) | <5 | 0 | 0 | 0 |
| RV14 | | RKGDI K S Y G L G P R Y G G | 0 | — | 0 | 0 | 0 |
| P73 | △ | S T K D L T T Y * G F G H Q N K A | 5(2) | — | 0 | 0 | 0 |
| P76 | ○ | G F G H Q N K A | 0 | 0 | 0 | 0 | 0 |
| P75 | △ | M Q K L L D T Y G I N L P L V T | 0 | — | — | 0 | 0 |
| P74 | ○ | G I N L P L V T | 0 | >15 | 10 | <10 | 0 |
| RV85 | | E R A S L TTA * GPSDMYVH | 48(18) | | | | |
| RV39 | | P R E N L TTA * GPSDMYVH | 11(5) | | | | |
| RV1A | | R R N T I TTA * GPSDMYVH | 10(4) | | | | |
| RV1B | | P R A S M K T V * GPSDMYVH | 10(4) | — | 0 | 0 | 0 |
| RV49 | | S R A I ITTA * GPSDMYVH | 53(23) | | | | |
| RV89 | | D V F T V T N V * GPS S M F VH | 0 | | | | |
| RV9 | | N V R A V K N V * GPSDMYVH | 0 | | | | |

**Verkürzte und deletierte Peptidsubstrate**

| # | | Sequenz | Gespalten (%) | 1:4 | 1:2 | 1:1 | 1:0.5 |
|---|---|---|---|---|---|---|---|
| P39 | △ | I ITTA GPS | 0 | — | — | 0 | 0 |
| P145 | △ | I ITTA GPSDM | 0 | 70 | — | 0 | 0 |
| WTC9 | △ | I ITTA GPSDM | 0 | — | 0 | 0 | 0 |
| WTD8 | △ | I ITTA GPSD | 0 | 55 | <5 | <5 | 0 |
| WT9 | △ | P IITTA GPS | 0 | 0 | 0 | 0 | 0 |
| WT10 | △ | P IITTA GPSD | 0 | 0 | 0 | 0 | — |
| WTN9 | △ | TRPIITTA * G | 9 | 0 | 0 | 0 | — |
| AB1 | | TRPIIT GPSDMYVH | 0 | — | — | <5 | 0 |
| AB2 | | TRPIITT PSDMYVH | 0 | 30 | 15 | <5 | 0 |
| ST2 | | RPIITT PSDMYVH | 0 | — | 5 | <5 | 0 |

**C-terminale Peptide von HRV2 2A**

| # | Sequenz | Gespalten (%) | 1:4 | 1:2 | 1:1 | 1:0.5 |
|---|---|---|---|---|---|---|
| P77 | GGDNHVAFIDLRHFHCAEEQ | 0 | — | 0 | 0 | — |

# Fig. 9

## Tabelle 3A

| Bezeichung | Sequenz [a] | Relative Spaltungs- [b] effizienz $(V_{max}/K_m)rel$ |
|---|---|---|
| | P1*P1' | |
| P89 | TRPIITTA*GPSDMYVH | 1.00 |
| E9 | TRPIITTA*GPSDMYV | 0.96 |
| F6 | TRPIITTv*GPSDMYV | 0.03 |
| F2 | TRPIITTI*GPSDMYV | 0.38 |
| F8 | TRPIITTp*GPSDMYV | keine Spaltung |
| H12 | TRPIITTf*GPSDMYV | 0.79 |
| F10 | TRPIITTm*GPSDMYV | 5.10 |
| F12 | TRPIITTt*GPSDMYV | 0.68 |
| H2 | TRPIITTy*GPSDMYV | 1.38 |
| G5 | TRPIITTn*GPSDMYV | 0.10 |
| G7 | TRPIITTq*GPSDMYV | 0.11 |
| G11 | TRPIITTd*GPSDMYV | keine Spaltung |
| G9 | TRPIITTe*GPSDMYV | keine Spaltung |
| H6 | TRPIITTr*GPSDMYV | 0.56 |
| H4 | TRPIITTk*GPSDMYV | 0.12 |
| A5 | TRPIITaA*GPSDMYV | keine Spaltung |
| A3 | TRPIITvA*GPSDMYV | keine Spaltung |
| A7 | TRPIITlA*GPSDMYV | keine Spaltung |
| A9 | TRPIITjA*GPSDMYV | keine Spaltung |
| A11 | TRPIITpA*GPSDMYV | 0.06 |
| D2 | TRPIITfA*GPSDMYV | keine Spaltung |

# Fig. 10

## Tabelle 3B

| Bezeichung | Sequenz [a) | Relative Spaltungs-[b) effizienz $(V_{max}/K_m)$rel |
|---|---|---|
| A1 | TRPIITgA*GPSDMYV | keine Spaltung |
| B4 | TRPIITsA*GPSDMYV | 0.15 |
| C7 | TRPIITyA*GPSDMYV | keine Spaltung |
| B8 | TRPIITnA*GPSDMYV | 0.10 |
| B10 | TRPIITqA*GPSDMYV | 0.03 |
| C1 | TRPIITdA*GPSDMYV | keine Spaltung |
| B12 | TRPIITeA*GPSDMYV | keine Spaltung |
| C3 | TRPIITkA*GPSDMYV | 0.05 |
| C5 | TRPIITrA*GPSDMYV | 0.16 |
| B6 | TRPIIThA*GPSDMYV | 0.04 |
| D4 | TRPIITTA*fPSDMYV | keine Spaltung |
| D10 | TRPIITTA*tPSDMYV | keine Spaltung |
| D6 | TRPIITTA*dPSDMYV | keine Spaltung |
| D8 | TRPIITTA*kPSDMYV | keine Spaltung |
| D12 | TRPIITTA*GfSDMYV | 0.28 |
| E5 | TRPIITTA*GtSDMYV | 0.37 |
| E1 | TRPIITTA*GdSDMYV | 0.22 |
| E3 | TRPIITTA*GkSDMYV | 0.43 |
| E7 | TRPIITTA*GPtDMYV | 0.74 |
| C9 | TRPIITTA*GPdDMYV | 0.23 |
| H8 | TRPIITTA*GPkDMYV | 0.76 |

# Fig. 11

# Tabelle 4

| Bezeichung [a) | Sequenz [b) | Relative Spaltungs- [c) effizienz $(V_{max}/K_m)$rel |
|---|---|---|
| | P1*P1' | |
| P89 | TRPIITTA*GPSDMYVH | 1.00 |
| RV49˙ | sRaIITTA*GPSDMYVH | 0.30 |
| RV85 | eRaslTTA*GPSDMYVH | 0.31 |
| RV1A˙ | rRntITTA*GPSDMYVH | 0.01 |
| RV39˙ | pRenlTTA*GPSDMYVH | 0.04 |
| P73 | slkdlTTy*Ggfhqnka | 0.02 |
| RV14 | rkgdlksy*GlgprYgg | keine Spaltung |
| RV1B˙ | pRasmkTV*GPSDMYVH | 0.03 |
| RV9 | nvravknv*GPSDMYVH | keine Spaltung |
| RV89 | dvftvTnv*GPSsMfVH | keine Spaltung |

Europäisches
Patentamt

EUROPÄISCHER RECHERCHENBERICHT

Nummer der Anmeldung

EP    92 11 8880
PAGE1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | VIROLOGY<br>Bd. 169, Nr. 1, 1989, NEW YORK US<br>Seiten 68 - 77<br>SOMMERGRUBER, WOLFGANG; ZORN, MANFRED;<br>BLAAS, DIETER; FESSL, FRIEDERIKE;<br>VOLKMANN, PETER; MAURER-FOGY, INGRID;<br>PALLAI, PETER; 'Polypeptide 2A of human<br>rhinovirus type 2: identification as a<br>protease and characterization by<br>mutational analysis' | 1,4,6,8,<br>17-19 | C12N9/50<br>C07K15/00<br>C12N15/57<br>A61K37/64 |
| A | * das ganze Dokument *<br>--- | 2 | |
| Y | WO-A-9 100 348 (BOERHINGER INGELHEIM<br>INTERNATIONAL GMBH) | 8,17-19 | |
| A | * Seite 14, Zeile 4 - Seite 22, Zeile 34;<br>Abbildung 1 *<br>--- | 1,4,6,11 | |
| Y | EP-A-0 196 921 (BIOTECHNOLOGY RESEARCH<br>ASSOCIATES J.V.) | 1,4,6 | |
| A | * Anspruch A *<br>--- | 5 | |
| A | EP-A-0 321 973 (BOERHINGER INGELHEIM<br>INTERNATIONAL)<br>* das ganze Dokument *<br>--- | 1,2,5,8 | RECHERCHIERTE SACHGEBIETE (Int. Cl.5)<br><br>C12N<br>C07K |
| A | CHEMICAL ABSTRACTS, vol. 110, no. 3<br>Columbus, Ohio, US;<br>abstract no. 20354m,<br>BILLICH, SUSANNE; KNOOP, MARIE THERES;<br>HANSEN, JUTTA; STROP, PETER; SEDLACEK,<br>JURAJ; MERTZ, RONALD; MOELLING, KARIN<br>'Synthetic peptides as substrates and<br>inhibitors of human immune deficiency<br>virus -1 protease'<br>& J. Biol. Chem., 263(34), 17905-8 1988<br>* Zusammenfassung *<br>---<br>-/-- | 15 | |

Der vorliegende Recherchenbericht wurde für alle Patentansprüche erstellt

| Recherchenort | Abschlußdatum der Recherche | Prüfer |
|---|---|---|
| BERLIN | 01 FEBRUAR 1993 | GURDJIAN D. |

Europäisches
Patentamt

**EUROPÄISCHER RECHERCHENBERICHT**

Nummer der Anmeldung

EP    92 11 8880
PAGE2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DATABASE WPIL<br>Week 8135,<br>Derwent Publications Ltd., London, GB;<br>AN 81-63212D [35]<br>& JP-A-56 086 142 (NIPPON KAYAKU KK) 13.<br>Juli 1981<br>* Zusammenfassung *<br>--- | 9,15 | |
| A | WO-A-8 604 901 (SANOFI)<br>* Ansprüche 1-12 *<br><br>----- | 9,14 | |
| | | | **RECHERCHIERTE SACHGEBIETE (Int. Cl.5)** |

Der vorliegende Recherchenbericht wurde für alle Patentansprüche erstellt

| Recherchenort | Abschlußdatum der Recherche | Prüfer |
|---|---|---|
| BERLIN | 01 FEBRUAR 1993 | GURDJIAN D. |